# EUROPEAN PATENT APPLICATION

(11) **EP 3 597 271 A1**
(43) Date of publication of application: **22.01.2020**
(21) Application number: 19176040.4
(22) Date of filing: 08.01.2016
(51) Int. Cl.: A61P 1/16, C07D 495/04, A61K 31/519, A61K 45/06

(54) **ACC INHIBITOR COMBINATION THERAPY FOR THE TREATMENT OF NON-ALCOHOLIC FATTY LIVER DISEASE**

(30) Priority: 09.01.2015 US 201562101726 P
(62) Divisional of application: 16735485.1
(71) Applicant: Gilead Apollo, LLC, Foster City CA 94404 (US)
(72) Inventor: HARRIMAN, Geraldine C., Charlestown, Rhode Island 02813 (US); KAPELLER-LIBERMANN, Rosana, Chestnut Hill, Massachusetts 02467 (US); WESTLIN, William F., Boxborough, Massachusetts 01719 (US); HARWOOD, H. James, Ledyard, Connecticut 06339 (US)
(74) Representative: Nieuwenhuys, William Francis

(57) **Abstract**

The present invention provides methods of treating, stabilizing or lessening the severity or progression of a non-alcoholic fatty liver disease using an ACC inhibitor alone or with one or more additional therapeutic agents.

## Description

### FIELD OF THE INVENTION

The present invention provides drug combinations and methods of treating, stabilizing or lessening the severity or progression of a non-alcoholic fatty liver disease (NAFLD).

This application is a divisional application filed from European patent application no. 16735485.1.

### BACKGROUND OF THE INVENTION

Non-alcoholic fatty liver disease (NAFLD) consists of a spectrum of conditions ranging from relatively benign steatosis to more severe non-alcoholic steatohepatitis (NASH), the latter of which can lead to fibrosis, cirrhosis, liver failure, or hepatocellular carcinoma if untreated. NAFLD is the most common cause of chronic liver disease in the United States, and is closely associated with obesity, type 2 diabetes, and metabolic syndrome.

The resistance of adipose tissue to insulin is believed to be one of the primary mechanisms resulting in the increased hepatic influx of non-esterified fatty acids (NEFA). In addition, lipogenesis and dietary intake also contribute to the accumulation of hepatic lipids. This increased heptatic fatty acid load is believed to be hepatotoxic, either because of the presence of toxic lipid intermediates or by causing oxidative stress and increased lipid peroxidation.

There has been significant interest in developing pharmacological agents to treat NASH. Insulin sensitizers such as metformin, thiazolidinediones such as rosiglitazone and pioglitazone, and antioxidants such as vitamin E have been evaluated in clinical trials. However, to date there are no approved treatments for NASH in the United States. Accordingly, there remains a need to find new treatments for this class of disorders.

### SUMMARY OF THE INVENTION

In some embodiments, the present invention provides methods of treating, stabilizing or lessening the severity or progression of a non-alcoholic fatty liver disease comprising administering to a patient in need thereof an inhibitor of Acetyl-CoA carboxylase (ACC) alone, or in combination with one or more additional therapeutic agents. In some aspects, the inhibitor of ACC has the general formula **I, II, III, IV,** or **V**: 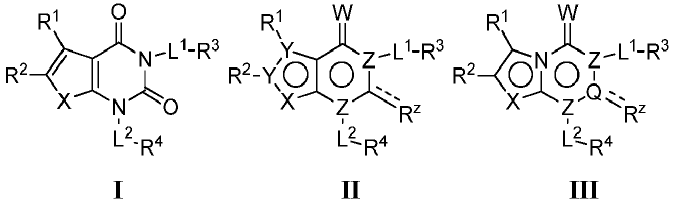 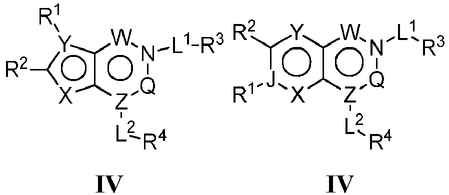 or a pharmaceutically acceptable salt thereof, wherein each variable is as defined and described herein.

In some embodiments, the present invention provides a pharmaceutical composition comprising an ACC inhibitor, one or more additional therapeutic agents, and a pharmaceutically acceptable carrier, adjuvant, or vehicle.

Additional embodiments describing methods of utilizing a provided combination are described in detail herein, *infra.*

### DETAILED DESCRIPTION OF THE INVENTION

As described herein, in some embodiments, the present invention provides methods of treating, stabilizing or lessening the severity or progression of a non-alcoholic fatty liver disease (NAFLD) comprising administering to a patient in need thereof an ACC inhibitor alone or in combination with one or more additional therapeutic agents.

In some embodiments, an ACC inhibitor is a compound of one of formulas **I, II, III, IV,** or **V,** or a pharmaceutically acceptable salt thereof, as described herein. In some embodiments, an ACC inhibitor is soraphen A.

In some embodiments, an additional therapeutic agent is a compound as described herein, *infra.*

### Definitions

As used herein generally, "ACC inhibitor" means any therapeutic agent that reduces the activity of an acetyl CoA carboxylase enzyme.

As used herein, "non-alcoholic fatty liver disease" or "NAFLD" means any disease or other deleterious condition characterized by, and/or caused by, excess hepatic fat accumulation, including, but not limited to, steatosis, non-alcoholic steatohepatitis (NASH), liver fibrosis caused by NASH, liver cirrhosis caused by NASH, or hepatocellular carcinoma (HCC) caused by NASH.

The term "subject", as used herein, means a mammal and includes human and animal subjects, such as domestic animals (e.g., horses, dogs, cats, etc.).

As used herein, a "therapeutically effective amount" means an amount of a substance (*e.g.,* a therapeutic agent, composition, and/or formulation) that elicits a desired biological response. In some embodiments, a therapeutically effective amount of a substance is an amount that is sufficient, when administered as part of a dosing regimen to a subject suffering from or susceptible to a disease, condition, or disorder, to treat, diagnose, prevent, and/or delay the onset of the disease, condition, or disorder. As will be appreciated by those of ordinary skill in this art, the effective amount of a substance may vary depending on such factors as the desired biological endpoint, the substance to be delivered, the target cell or tissue, *etc.* For example, the effective amount of compound in a formulation to treat a disease, condition, or disorder is the amount that alleviates, ameliorates, relieves, inhibits, prevents, delays onset of, reduces severity of and/or reduces incidence of one or more symptoms or features of the disease, condition, or disorder. In some embodiments, a "therapeutically effective amount" is at least a minimal amount of a compound, or composition containing a compound, which is sufficient for treating one or more symptoms of a NAFLD.

The terms "treat" or "treating," as used herein, refers to partially or completely alleviating, inhibiting, delaying onset of, preventing, ameliorating and/or relieving a disease or disorder, or one or more symptoms of the disease or disorder. As used herein, the terms "treatment," "treat," and "treating" refer to partially or completely alleviating, inhibiting, delaying onset of, preventing, ameliorating and/or relieving a disease or disorder, or one or more symptoms of the disease or disorder, as described herein. In some embodiments, treatment may be administered after one or more symptoms have developed. In some embodiments, the term "treating" includes preventing or halting the progression of a disease or disorder. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence. Thus, in some embodiments, the term "treating" includes preventing relapse or recurrence of a disease or disorder.

The expression "unit dosage form" as used herein refers to a physically discrete unit of therapeutic formulation appropriate for the subject to be treated. It will be understood, however, that the total daily usage of the compositions of the present invention will be decided by the attending physician within the scope of sound medical judgment. The specific effective dose level for any particular subject or organism will depend upon a variety of factors including the disorder being treated and the severity of the disorder; activity of specific active agent employed; specific composition employed; age, body weight, general health, sex and diet of the subject; time of administration, and rate of excretion of the specific active agent employed; duration of the treatment; drugs and/or additional therapies used in combination or coincidental with specific compound(s) employed, and like factors well known in the medical arts.

### ACC Inhibitors

As described generally above, in some embodiments, the present invention provides methods of treating, stabilizing or lessening the severity or progression of a NAFLD comprising administering to a patient in need thereof an ACC inhibitor alone, or in combination with one or more additional therapeutic agents. In some embodiments, the ACC inhibitor has the general formula **I:** 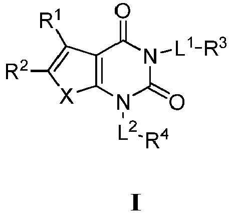 or a pharmaceutically acceptable salt thereof, wherein:
X is -O-, -S-, or -NR-;
R¹ is hydrogen or C₁₋₄ aliphatic, optionally substituted with one or more halogen, -OR, -SR, -N(R)₂, -N(R)C(O)R, -C(O)N(R)₂, -N(R)C(O)N(R)₂, -N(R)C(O)OR, -OC(O)N(R)₂, -N(R)SO₂R,-SO₂N(R)₂, -C(O)R, -C(O)OR, -OC(O)R, -S(O)R, or -SO₂R;
R² is halogen, -R, -OR, -SR, -N(R)₂, -N(R)C(O)R, -C(O)N(R)₂, -N(R)C(O)N(R)₂,-N(R)C(O)OR, -OC(O)N(R)₂, -N(R)SO₂R, -SO₂N(R)₂, -C(O)R, -C(O)OR, -OC(O)R, -S(O)R, or -SO₂R, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
or R¹ and R² are taken together to form an optionally substituted 4-7 membered partially unsaturated carbocyclo-, or heterocyclo-, benzo-, or 5-6 membered heteroarylo- fused ring;
each R is independently hydrogen or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
each of L¹ and L² is independently a covalent bond or an optionally substituted 1-6 membered straight or branched bivalent hydrocarbon chain; or a cyclopropylenyl, cyclobutylenyl, or oxetanyl group;
R³ is hydrogen, halogen, -CN,-OR, -SR, -N(R)₂, -N(R)C(O)R, -C(O)N(R)₂, -C(O)N(R)S(O)₂R,-N(R)C(O)N(R)₂, -N(R)C(O)OR, -OC(O)N(R)₂, -N(R)SO₂R, -SO₂N(R)₂, -C(O)R,-C(O)OR, -OC(O)R, -S(O)R, -SO₂R, -B(OH)₂, or an optionally substituted ring selected from phenyl or 5-6 membered heteroaryl having 1-4 heteroatoms independently selected from nitrogen, oxygen, or sulfur; and
R⁴ is hydrogen or an optionally substituted ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur.

In some embodiments, the ACC inhibitor of formula **I** is a compound of formula **I-a:** 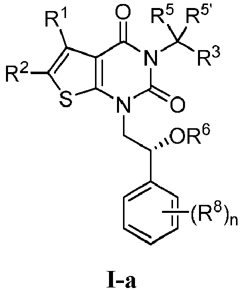 or a pharmaceutically acceptable salt thereof, wherein:
R¹ is hydrogen or C₁₋₄ aliphatic, optionally substituted with one or more halogen, -OR, -SR, -N(R)₂, -N(R)C(O)R, -C(O)N(R)₂, -N(R)C(O)N(R)₂, -N(R)C(O)OR, -OC(O)N(R)₂, -N(R)SO₂R,-SO₂RN(R)₂, -C(O)R, -C(O)OR, -OC(O)R, -C(O)OR, -S(O)R, or -SO₂R;
R² is halogen, -R, -OR, -SR, -N(R)₂, -N(R)C(O)R, -C(O)N(R)₂, -N(R)C(O)N(R)₂,-N(R)C(O)OR, -OC(O)N(R)₂, -N(R)SO₂R, -SO₂N(R)₂, -C(O)R, -C(O)OR, -OC(O)R, -S(O)R, or -SO₂R; or R¹ and R² are taken together to form an optionally substituted 4-7 membered partially unsaturated carbocyclo-, or heterocyclo-, benzo-, or 5-6 membered heteroarylo- fused ring;
each R is independently hydrogen or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
R³ is hydrogen, halogen, -CN, -OR, -SR, -N(R)₂, -N(R)C(O)R, -C(O)N(R)₂, -N(R)C(O)N(R)₂,-N(R)C(O)OR, -OC(O)N(R)₂, -N(R)SO₂R, -SO₂N(R)₂, -C(O)R, -C(O)OR, -OC(O)R,-S(O)R, -SO₂R, -B(OH)₂, or an optionally substituted ring selected from phenyl or 5-6 membered heteroaryl having 1-4 heteroatoms independently selected from nitrogen, oxygen, or sulfur;
each of R⁵ and R⁵' is independently -R, -OR, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, or -SO₂R or R⁵ and R⁵' are taken together to form a cyclopropylenyl, cyclobutylenyl, or oxetanyl group; and
R⁶ is -R, -C(O)N(R)₂, or -C(O)R;
each R⁸ is independently selected from halogen, -R, -OR, -SR, -N(R)₂ or deuterium; and n is 0-5.

Suitable ACC inhibitors of formula **I** include those described in WO2013/071169A1, referred to herein as "the '169 application," the entirety of which is hereby incorporated by reference. The ACC inhibitors of formula **I** are active in a variety of assays and therapeutic models demonstrating inhibition of one or both of ACC1 and ACC2 enzymes (see, e.g., Table 2 of the '169 application).

In some embodiments, the ACC inhibitor of formula **I** is **1-181** or **1-278:** 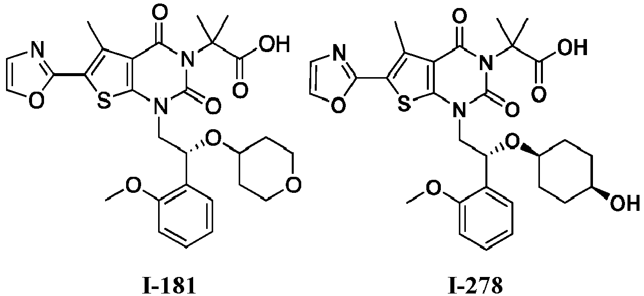 or a pharmaceutically acceptable salt thereof. The syntheses of compounds **1-181** and **1-278** are described in paragraphs [00526] - [00532] and [00680] - [00681] of the '169 application respectively. In some embodiments, the ACC inhibitor of formula **I** is **1-181,** or a pharmaceutically acceptable salt thereof. In some embodiments, the ACC inhibitor of formula **I** is **1-278,** or a pharmaceutically acceptable salt thereof.

As described in the ' 169 application, compounds of formula I are potent inhibitors of the ACC enzymes, including ACC1 and ACC2. In some embodiments, the ACC inhibitor of formula I inhibits one or both of ACC1 and ACC2 with an IC₅₀ ≤ 0.1 µM, ≤ 1 µM, ≤ 5 µM, 5-20 µM, 20-50 µM, or ≥ 50 µM. Most preferably, the ACC inhibitor of formula I inhibits one or both of ACC1 and ACC2 with an IC₅₀ ≤ 0.1 µM.

As described in the ' 169 application, ACC inhibitors of formula I have demonstrated potent *in vitro* and *in vivo* ability to decrease the rate of radiolabeled [¹⁴C] acetate into the fatty acids of liver cells (see Table 4 and Figure 8 of the '169 application for *in vivo* and *in vitro* data respectively).

In some embodiments, the ACC inhibitor has the general formula **II**: 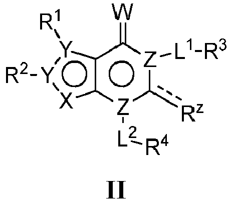 or a pharmaceutically acceptable salt thereof, wherein:
W is oxygen or sulfur;
X is O, S, N or NR;
each Y is independently C, N, or O;
each Z is independently C or N;
R¹ is hydrogen or C₁₋₄ aliphatic, optionally substituted with one or more halogens, -OR, -SR,-N(R)₂, -N(R)C(O)R, -C(O)N(R)₂, -N(R)C(O)N(R)₂, -N(R)C(O)OR, -OC(O)N(R)₂,-N(R)SO₂R, -SO₂RN(R)₂, -C(O)R, -C(O)OR, -OC(O)R, -C(O)OR, -S(O)R, or -SO₂R;
R² is halogen, -R, -OR, -SR, -N(R)₂, -N(R)C(O)R,-C(O)N(R)₂, -N(R)C(O)N(R)₂, -N(R)C(O)OR, -OC(O)N(R)₂, -N(R)SO₂R,-SO₂N(R)₂, -C(O)R, -C(O)OR, -OC(O)R, -S(O)R, -SO₂R, -B(OR)₂, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; wherein R² is not optionally substituted benzyl; or
R¹ and R² are taken together to form an optionally substituted 4-7 membered partially unsaturated carbocyclo-, or heterocyclo-, benzo-, or 5-6 membered heteroarylo- fused ring;
each R is independently hydrogen, deuterium, or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
L¹ is a covalent bond or a 1-6 membered straight or branched bivalent hydrocarbon chain optionally substituted with R⁵ and R^{5'};
L² is a covalent bond or a 1-6 membered straight or branched bivalent hydrocarbon chain optionally substituted with R⁷ and R⁷;
R³ is halogen, -CN, -OR, -SR, -N(R)₂, -N(R)C(O)R, -C(O)RN(R)₂, -C(O)N(R)S(O)₂R,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
each of R⁵ and R⁵' is independently -R, -OR, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, or -SO₂R; or R⁵ and R⁵ are taken together to form a cyclopropylenyl, cyclobutylenyl, or oxetanyl group;
each of R⁷ and R^{7'} is independently, -R, -OR⁶, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
R⁶ is -R, -C(O)N(R)₂, or -C(O)R;
each R⁸ is independently selected from halogen, -R, -OR, -SR, -N(R)₂ or deuterium;
R^{z} is selected from hydrogen, halogen, methyl, -CN, =O, and =S; and
n is 0-5.

Suitable ACC inhibitors of formula **II** include those described in WO2014/182943A1, referred to herein as "the '943 application," the entirety of which is hereby incorporated by reference. The ACC inhibitors of formula **II** are active in a variety of assays and therapeutic models demonstrating inhibition of one or both of ACC1 and ACC2 enzymes (see, e.g., paragraphs [00309] - [00317] and Table 2 of the '943 application).

In some embodiments, the ACC inhibitor has the general formula **III:** 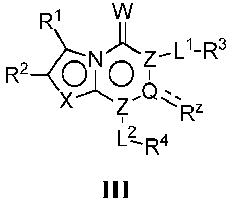 or a pharmaceutically acceptable salt thereof, wherein:
W is oxygen or sulfur;
Q is C or N; wherein if Q is N, then R^{Z} is absent;
X is -O-, -S-, or -NR-;
each Z is independently C or N; provided that both Z are not N;
R¹ is hydrogen or C₁₋₄ aliphatic, optionally substituted with one or more halogens, -OR, -SR,-N(R)₂, -N(R)C(O)R, -C(O)N(R)₂, -N(R)C(O)N(R)₂, -N(R)C(O)OR, -OC(O)N(R)₂,-N(R)SO₂R, -SO₂RN(R)₂, -C(O)R, -C(O)OR, -OC(O)R, -C(O)OR, -S(O)R, or -SO₂R;
R² is halogen, -R, -OR, -SR, -N(R)₂, -N(R)C(O)R, -C(O)N(R)₂, -N(R)C(O)N(R)₂,-N(R)C(O)OR, -OC(O)N(R)₂, -N(R)SO₂R, -SO₂N(R)₂, -C(O)R, -C(O)OR, -OC(O)R,-S(O)R, -SO₂R, -B(OR)₂, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; or
R¹ and R² are taken together to form an optionally substituted 4-7 membered partially unsaturated carbocyclo-, or heterocyclo-, benzo-, or 5-6 membered heteroarylo- fused ring;
each R is independently hydrogen, deuterium, or an optionally substituted group selected from C_{1- 6} aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
L¹ is a covalent bond or a 1-6 membered straight or branched bivalent hydrocarbon chain optionally substituted with R⁵ and R^{5'};
L² is a covalent bond or a 1-6 membered straight or branched bivalent hydrocarbon chain optionally substituted with R⁷ and R^{7'};
R³ is halogen, -CN, -OR, -SR, -N(R)₂, -N(R)C(O)R, -C(O)RN(R)₂, -C(O)N(R)S(O)₂R,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
each of R⁵ and R⁵' is independently -R, -OR, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, or -SO₂R; or R⁵ and R⁵ are taken together to form a cyclopropylenyl, cyclobutylenyl, or oxetanyl group; and
each of R⁷ and R^{7'} is independently, -R, -OR⁶, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
R⁶ is -R, -C(O)N(R)₂, or -C(O)R;
each R⁸ is independently selected from halogen, -R, -OR, -SR, -N(R)₂ or deuterium;
R^{Z} is selected from hydrogen, halogen, methyl, -CN, =O, and =S; and
n is 0-5.

Suitable ACC inhibitors of formula III include those described in WO2014/182945A1, referred to herein as "the '945 application," the entirety of which is hereby incorporated by reference. The ACC inhibitors of formula III are active in a variety of assays and therapeutic models demonstrating inhibition of one or both of ACC1 and ACC2 enzymes (see, e.g., paragraphs [00382] - [00304] of the '945 application).

In some embodiments, the ACC inhibitor has the general formula IV: 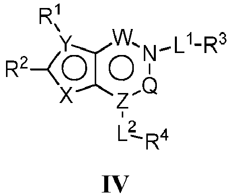 or a pharmaceutically acceptable salt thereof, wherein:
W is -C(O)-, -C(S)-, or -S(O)₂-;
Q is -C(O)-, -C(S)-, -S(O)₂-, or N;
X is -O-, -S-, -NR-, or N;
Y is C or N;
Z is C or N;
R¹ is hydrogen or C₁₋₄ aliphatic, optionally substituted with one or more halogens, -OR, -SR,-N(R)₂, -N(R)C(O)R, -C(O)N(R)₂, -N(R)C(O)N(R)₂, -N(R)C(O)OR, -OC(O)N(R)₂,-N(R)SO₂R, -SO₂RN(R)₂, -C(O)R, -C(O)OR, -OC(O)R, -C(O)OR, -S(O)R, or -SO₂R;
R² is halogen, -R, -OR, -SR, -N(R)₂, -N(R)C(O)R,-C(O)N(R)₂, -N(R)C(O)N(R)₂, -N(R)C(O)OR, -OC(O)N(R)₂, -N(R)SO₂R,-SO₂N(R)₂, -C(O)R, -C(O)OR, -OC(O)R, -S(O)R, or -SO₂R, -B(OR)₂, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; wherein R² is not optionally substituted benzyl; or
R¹ and R² are taken together to form an optionally substituted 4-7 membered partially unsaturated carbocyclo-, or heterocyclo-, benzo-, or 5-6 membered heteroarylo- fused ring;
each R is independently hydrogen, deuterium, or an optionally substituted group selected from C_{1- 6} aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
L¹ is a covalent bond or a 1-6 membered straight or branched bivalent hydrocarbon chain optionally substituted with R⁵ and R^{5'};
L² is a covalent bond or a 1-6 membered straight or branched bivalent hydrocarbon chain optionally substituted with R⁷ and R⁷;
R³ is halogen, -CN, -OR, -SR, -N(R)₂, -N(R)C(O)R, -C(O)RN(R)₂, -C(O)N(R)S(O)₂R,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
each of R⁵ and R⁵' is independently -R, -OR, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, or -SO₂R; or R⁵ and R⁵ are taken together to form a cyclopropylenyl, cyclobutylenyl, or oxetanyl group;
each of R⁷ and R^{7'} is independently, -R, -OR⁶, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, or -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
R⁶ is -R, -C(O)N(R)₂, or -C(O)R;
each R⁸ is independently selected from halogen, -R, -OR, -SR, -N(R)₂ or deuterium; and n is 0-5.

Suitable ACC inhibitors of formula **IV** include those described in WO2014/182950A1, referred to herein as "the '950 application," the entirety of which is hereby incorporated by reference. The ACC inhibitors of formula **IV** are active in a variety of assays and therapeutic models demonstrating inhibition of one or both of ACC1 and ACC2 enzymes (see, e.g., paragraphs [00336] - [00344] and Table 2 of the '950 application).

In some embodiments, the ACC inhibitor has the formula **V:** 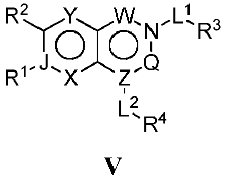 or a pharmaceutically acceptable salt thereof, wherein:
W is -C(R^{Z})-, -C(O)-, or -C(S)-;
Q is -C(R^{Z})-, -C(O)-, or -C(S)-;
J is C or N; provided that when J is N, R¹ is absent;
X is CH or N;
Y is CH or N;
Z is C or N;
R¹ is hydrogen or C₁₋₄ aliphatic, optionally substituted with one or more halogens, -OR, -SR,-N(R)₂, -N(R)C(O)R, -C(O)N(R)₂, -N(R)C(O)N(R)₂, -N(R)C(O)OR, -OC(O)N(R)₂,-N(R)SO₂R, -SO₂RN(R)₂, -C(O)R, -C(O)OR, -OC(O)R, -C(O)OR, -S(O)R, or -SO₂R;
R² is halogen, -R, -OR, -SR, -N(R)₂, -N(R)C(O)R, -C(O)N(R)₂, -N(R)C(O)N(R)₂,-N(R)C(O)OR, -OC(O)N(R)₂, -N(R)SO₂R, -SO₂N(R)₂, -C(O)R, -C(O)OR, -OC(O)R,-S(O)R, -B(OR)₂, -SO₂R or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; or
R¹ and R² are taken together to form an optionally substituted 4-7 membered partially unsaturated carbocyclo-, or heterocyclo-, benzo-, or 5-6 membered heteroarylo- fused ring;
each R is independently hydrogen, deuterium, or an optionally substituted group selected from C_{1- 6} aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
L¹ is a covalent bond or a 1-6 membered straight or branched bivalent hydrocarbon chain optionally substituted with R⁵ and R^{5'};
L² is a covalent bond or a 1-6 membered straight or branched bivalent hydrocarbon chain optionally substituted with R⁷ and R^{7'};
R³ is hydrogen, halogen, -CN, -OR, -SR, -N(R)₂, -N(R)C(O)R, -C(O)RN(R)₂, -C(O)N(R)S(O)₂R, -N(R)C(O)N(R)₂, -N(R)C(O)OR, -OC(O)N(R)₂, -N(R)SO₂R, -SO₂N(R)₂, -C(O)R, -C(O)OR, -OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
each of R⁵ and R⁵' is independently -R, -OR, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, or -SO₂R; or R⁵ and R⁵ are taken together to form a cyclopropylenyl, cyclobutylenyl, or oxetanyl group;
each of R⁷ and R^{7'} is independently, -R, -OR⁶, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, or -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
R⁶ is -R, -C(O)N(R)₂, or -C(O)R;
each R⁸ is independently selected from halogen, -R, -OR, -SR, -N(R)₂ or deuterium;
each R^{Z} is independently selected from the group consisting of hydrogen, halogen, C₁-C₃ alkyl, and -CN; and
n is 0-5.

Suitable ACC inhibitors of formula **V** include those described in WO2014/182951A1, referred to herein as "the '951 application," the entirety of which is hereby incorporated by reference. The ACC inhibitors of formula **V** are active in a variety of assays and therapeutic models demonstrating inhibition of one or both of ACC1 and ACC2 enzymes (see, e.g., paragraphs [00295] - [00326] and Table 2 of the '951 application).

### Additional therapeutic agents

As described generally above, provided methods comprise combination therapies utilizing an ACC inhibitor and one or more additional therapeutic agents. In some embodiments, provided methods comprise administering an ACC inhibitor with one additional therapeutic agent. In some embodiments, provided methods comprise administering an ACC inhibitor with two additional therapeutic agents. In some embodiments, provided methods comprise administering an ACC inhibitor with three additional therapeutic agents.

In some embodiments, the present invention provides a method of treating, stabilizing or lessening the severity or progression of a NAFLD, comprising administering to a patient in need thereof an ACC inhibitor, in combination with one or more additional therapeutic agents. In certain embodiments, the one or more additional therapeutic agents are independently selected from the group consisting of angiotensin II receptor antagonists, angiotensin converting enzyme (ACE) inhibitors, caspase inhibitors, cathepsin B inhibitors, CCR2 chemokine antagonists, CCR5 chemokine antagonists, chloride channel stimulators, cholesterol solubilizers, diacylglycerol O-acyltransferase 1 (DGAT1) inhibitors, dipeptidyl peptidase IV (DPPIV) inhibitors, farnesoid X receptor (FXR) agonists, FXR/TGR5 dual agonists, galectin-3 inhibitors, glucagon-like peptide 1

(GLP1) agonists, glutathione precursors, hepatitis C virus NS3 protease inhibitors, HMG CoA reductase inhibitors, 11β-hydroxysteroid dehydrogenase (11β-HSD1) inhibitors, IL-1β antagonists, IL-6 antagonists, IL-10 agonists, IL-17 antagonists, ileal sodium bile acid cotransporter inhibitors, leptin analogs, 5-lipoxygenase inhibitors, LPL gene stimulators, lysyl oxidase homolog 2 (LOXL2) inhibitors, PDE3 inhibitors, PDE4 inhibitors, phospholipase C (PLC) inhibitors, PPARα agonists, PPARγ agonists, PPARδ agonists, Rho associated protein kinase 2 (ROCK2) inhibitors, sodium glucose transporter-2 (SGLT2) inhibitors, stearoyl CoA desaturase-1 inhibitors, thyroid hormone receptor β agonists, tumor necrosis factor α (TNFα) ligand inhibitors, transglutaminase inhibitors, transglutaminase inhibitor precursors, PTPlb inhibitors, and ASK1 inhibitors.

In some embodiments, an ACC inhibitor is administered in combination with one or more additional therapeutic agents, wherein at least one of the additional therapeutic agents is an angiotensin II receptor antagonist.

In some embodiments, an ACC inhibitor is administered in combination with one or more additional therapeutic agents, wherein at least one of the additional therapeutic agents is an angiotensin converting enzyme (ACE) inhibitor. In some embodiments, the ACE inhibitor is enalapril.

In some embodiments, an ACC inhibitor is administered in combination with one or more additional therapeutic agents, wherein at least one of the additional therapeutic agents is a caspase inhibitor. In some embodiments the caspase inhibitor is emricasan.

In some embodiments, an ACC inhibitor is administered in combination with one or more additional therapeutic agents, wherein at least one of the additional therapeutic agents is a cathepsin B inhibitor. In some embodiments the cathepsin B inhibitor is a mixed cathepsin B/hepatitis C virus NS3 protease inhibitor. In some embodiments, the mixed cathepsin B/hepatitis C virus NS3 protease inhibitor is VBY-376.

In some embodiments, an ACC inhibitor is administered in combination with one or more additional therapeutic agents, wherein at least one of the additional therapeutic agents is a CCR2 chemokine antagonist. In some embodiments, the additional therapeutic agent is a mixed CCR2/CCR5 chemokine antagonist. In some embodiments, the mixed CCR2/CCR5 chemokine antagonist is cenicriviroc.

In some embodiments, an ACC inhibitor is administered in combination with one or more additional therapeutic agents, wherein at least one of the additional therapeutic agents is a CCR5 chemokine antagonist.

In some embodiments, an ACC inhibitor is administered in combination with one or more additional therapeutic agents, wherein at least one of the additional therapeutic agents is a chloride channel stimulator. In some embodiments, the chloride channel stimulator is cobiprostone.

In some embodiments, an ACC inhibitor is administered in combination with one or more additional therapeutic agents, wherein at least one of the additional therapeutic agents is a cholesterol solubilizer.

In some embodiments, an ACC inhibitor is administered in combination with one or more additional therapeutic agents, wherein at least one of the additional therapeutic agents is a diacylglycerol O-acyltransferase 1 (DGAT1) inhibitor. In some embodiments, the DGAT1 inhibitor is LCQ908.

In some embodiments, an ACC inhibitor is administered in combination with one or more additional therapeutic agents, wherein at least one of the additional therapeutic agents is a dipeptidyl peptidase IV (DPPIV) inhibitor. In some embodiments, the DPPIV inhibitor is linagliptin.

In some embodiments, an ACC inhibitor is administered in combination with one or more additional therapeutic agents, wherein at least one of the additional therapeutic agents is a farnesoid X receptor (FXR) agonist. In some embodiments, the FXR agonist is INT-747 (obeticholic acid). In some embodiments, the FXR agonist is PX-102.

In some embodiments, an ACC inhibitor is administered in combination with one or more additional therapeutic agents, wherein at least one of the additional therapeutic agents is an FXR/TGR5 dual agonist. In some embodiments, the FXR/TGR5 dual agonist is INT-767.

In some embodiments, an ACC inhibitor is administered in combination with one or more additional therapeutic agents, wherein at least one of the additional therapeutic agents is a galectin-3 inhibitor. In some embodiments, the galectin-3 inhibitor is GR-MD-02.

In some embodiments, an ACC inhibitor is administered in combination with one or more additional therapeutic agents, wherein at least one of the additional therapeutic agents is a glucagon-like peptide 1 (GLP1) agonist. In some embodiments, the GLP1 agonist is liraglutide. In some embodiments, the GLP1 agonist is exenatide.

In some embodiments, an ACC inhibitor is administered in combination with one or more additional therapeutic agents, wherein at least one of the additional therapeutic agents is a glutathione precursor.

In some embodiments, an ACC inhibitor is administered in combination with one or more additional therapeutic agents, wherein at least one of the additional therapeutic agents is a hepatitis C virus NS3 protease inhibitor. In some embodiments the heptatitis C virus NS3 protease inhibitor is a mixed cathepsin B/hepatitis C virus NS3 protease inhibitor. In some embodiments, the mixed cathepsin B/hepatitis C virus NS3 protease inhibitor is VBY-376.

In some embodiments, an ACC inhibitor is administered in combination with one or more additional therapeutic agents, wherein at least one of the additional therapeutic agents is an HMG CoA reductase inhibitor. In some embodiments, the HMG-CoA reductase inhibitor is a statin. In some embodiments, the HMG-CoA reductase inhibitor is atorvastatin.

In some embodiments, an ACC inhibitor is administered in combination with one or more additional therapeutic agents, wherein at least one of the additional therapeutic agents is an 11β-hydroxysteroid dehydrogenase (11β-HSD1) inhibitor. In some embodiments, the 11β-HSD1 inhibitor is RO5093151.

In some embodiments, an ACC inhibitor is administered in combination with one or more additional therapeutic agents, wherein at least one of the additional therapeutic agents is an IL-1β antagonist.

In some embodiments, an ACC inhibitor is administered in combination with one or more additional therapeutic agents, wherein at least one of the additional therapeutic agents is an IL-6 antagonist. In some embodiments, the IL-6 antagonist is a mixed IL-6/IL-1β/TNFα ligand inhibitor. In some embodiments, the mixed IL-6/IL-1β/TNFα ligand inhibitor is BLX-1002.

In some embodiments, an ACC inhibitor is administered in combination with one or more additional therapeutic agents, wherein at least one of the additional therapeutic agents is an IL-10 agonist. In some embodiments, the IL-10 agonist is peg-ilodecakin.

In some embodiments, an ACC inhibitor is administered in combination with one or more additional therapeutic agents, wherein at least one of the additional therapeutic agents is an IL-17 antagonist. In some embodiments, the IL-17 antagonist is KD-025.

In some embodiments, an ACC inhibitor is administered in combination with one or more additional therapeutic agents, wherein at least one of the additional therapeutic agents is an ileal sodium bile acid cotransporter inhibitor. In some embodiments, the ileal sodium bile acid cotransporter inhibitor is SHP-626.

In some embodiments, an ACC inhibitor is administered in combination with one or more additional therapeutic agents, wherein at least one of the additional therapeutic agents is a leptin analog. In some embodiments the leptin analog is metreleptin.

In some embodiments, an ACC inhibitor is administered in combination with one or more additional therapeutic agents, wherein at least one of the additional therapeutic agents is a 5-lipoxygenase inhibitor. In some embodiments, the 5-lipoxygenase inhibitor is a mixed 5-lipoxygenase/PDE3/PDE4/PLC inhibitor. In some embodiments, the mixed 5-lipoxygenase/PDE3/PDE4/PLC inhibitor is tipelukast.

In some embodiments, an ACC inhibitor is administered in combination with one or more additional therapeutic agents, wherein at least one of the additional therapeutic agents is a LPL gene stimulator. In some embodiments the LPL gene stimulator is alipogene tiparvovec.

In some embodiments, an ACC inhibitor is administered in combination with one or more additional therapeutic agents, wherein at least one of the additional therapeutic agents is a lysyl oxidase homolog 2 (LOXL2) inhibitor. In some embodiments, the LOXL2 inhibitor is an anti-LOXL2 antibody. In some embodiments, the anti-LOXL2 antibody is GS-6624.

In some embodiments, an ACC inhibitor is administered in combination with one or more additional therapeutic agents, wherein at least one of the additional therapeutic agents is a PDE3 inhibitor. In some embodiments, the PDE3 inhibitor is a mixed 5-lipoxygenase/PDE3/PDE4/PLC inhibitor. In some embodiments, the mixed 5-lipoxygenase/PDE3/PDE4/PLC inhibitor is tipelukast.

In some embodiments, an ACC inhibitor is administered in combination with one or more additional therapeutic agents, wherein at least one of the additional therapeutic agents is a PDE4 inhibitor. In some embodiments, the PDE4 inhibitor is ASP-9831. In some embodiments, the PDE4 inhibitor is a mixed 5-lipoxygenase/PDE3/PDE4/PLC inhibitor. In some embodiments, the mixed 5-lipoxygenase/PDE3/PDE4/PLC inhibitor is tipelukast.

In some embodiments, an ACC inhibitor is administered in combination with one or more additional therapeutic agents, wherein at least one of the additional therapeutic agents is a phospholipase C (PLC) inhibitor. In some embodiments, the PLC inhibitor is a mixed 5-lipoxygenase/PDE3/PDE4/PLC inhibitor. In some embodiments, the mixed 5-lipoxygenase/PDE3/PDE4/PLC inhibitor is tipelukast.

In some embodiments, an ACC inhibitor is administered in combination with one or more additional therapeutic agents, wherein at least one of the additional therapeutic agents is a PPARα agonist. In some embodiments the PPARα agonist is a mixed PPARα/δ agonist. In some embodiments, the mixed PPARα/δ agonist is GFT505.

In some embodiments, an ACC inhibitor is administered in combination with one or more additional therapeutic agents, wherein at least one of the additional therapeutic agents is a PPARγ agonist. In some embodiments, the PPARγ agonist is pioglitazone.

In some embodiments, an ACC inhibitor is administered in combination with one or more additional therapeutic agents, wherein at least one of the additional therapeutic agents is a PPARδ agonist.

In some embodiments, an ACC inhibitor is administered in combination with one or more additional therapeutic agents, wherein at least one of the additional therapeutic agents is a Rho associated protein kinase 2 (ROCK2) inhibitor. In some embodiments the ROCK2 inhibitor is KD-025.

In some embodiments, an ACC inhibitor is administered in combination with one or more additional therapeutic agents, wherein at least one of the additional therapeutic agents is a sodium glucose transporter-2 (SGLT2) inhibitor. In some embodiments, the SGLT2 inhibitor is remogliflozin etabonate.

In some embodiments, an ACC inhibitor is administered in combination with one or more additional therapeutic agents, wherein at least one of the additional therapeutic agents is a stearoyl CoA desaturase-1 inhibitor. In some embodiments, the stearoyl CoA desaturase-1 inhibitor is aramchol. In some embodiments, the stearoyl CoA desaturase-1 inhibitor is CVT-12805.

In some embodiments, an ACC inhibitor is administered in combination with one or more additional therapeutic agents, wherein at least one of the additional therapeutic agents is a thyroid hormone receptor β agonist. In some embodiments the thyroid hormone receptor β agonist is MGL-3196.

In some embodiments, an ACC inhibitor is administered in combination with one or more additional therapeutic agents, wherein at least one of the additional therapeutic agents is a tumor necrosis factor α (TNFα) ligand inhibitor.

In some embodiments, an ACC inhibitor is administered in combination with one or more additional therapeutic agents, wherein at least one of the additional therapeutic agents is a transglutaminase inhibitor. In some embodiments, the transglutaminase inhibitor precursor is mercaptamine.

In some embodiments, an ACC inhibitor is administered in combination with one or more additional therapeutic agents, wherein at least one of the additional therapeutic agents is a transglutaminase inhibitor precursor.

In some embodiments, an ACC inhibitor is administered in combination with one or more additional therapeutic agents, wherein at least one of the additional therapeutic agents is a PTPlb inhibitor. In some embodiments, the PTPlb inhibitor is A119505, A220435, A321842, CPT633, ISIS-404173, JTT-551, MX-7014, MX-7091, MX-7102, NNC-521246, OTX-001, OTX-002, or TTP814.

In some embodiments, an ACC inhibitor is administered in combination with one or more additional therapeutic agents, wherein at least one of the additional therapeutic agents is an ASK1 inhibitor. In some embodiments, the ASK1 inhibitor is GS4977.

In some embodiments, the one or more additional therapeutic agents are independently selected from acetylsalicylic acid, alipogene tiparvovec, aramchol, atorvastatin, BLX-1002, cenicriviroc, cobiprostone, colesevelam, emricasan, enalapril, GFT-505, GR-MD-02, hydrochlorothiazide, icosapent ethyl ester (ethyl eicosapentaenoic acid), IMM-124E, KD-025, linagliptin, liraglutide, mercaptamine, MGL-3196, obeticholic acid, olesoxime, peg-ilodecakin, pioglitazone, PX-102, remogliflozin etabonate, SHP-626, solithromycin, tipelukast, TRX-318, ursodeoxycholic acid, and VBY-376.

In some embodiments, one of the one or more additional therapeutic agents is acetylsalicylic acid. In some embodiments, one of the one or more additional therapeutic agents is alipogene tiparvovec. In some embodiments, one of the one or more additional therapeutic agents is aramchol. In some embodiments, one of the one or more additional therapeutic agents is atorvastatin. In some embodiments, one of the one or more additional therapeutic agents is BLX-1002. In some embodiments, one of the one or more additional therapeutic agents is cenicriviroc. In some embodiments, one of the one or more additional therapeutic agents is cobiprostone. In some embodiments, one of the one or more additional therapeutic agents is colesevelam. In some embodiments, one of the one or more additional therapeutic agents is emricasan. In some embodiments, one of the one or more additional therapeutic agents is enalapril. In some embodiments, one of the one or more additional therapeutic agents is GFT-505. In some embodiments, one of the one or more additional therapeutic agents is GR-MD-02. In some embodiments, one of the one or more additional therapeutic agents is hydrochlorothiazide. In some embodiments, one of the one or more additional therapeutic agents is icosapent ethyl ester (ethyl eicosapentaenoic acid). In some embodiments, one of the one or more additional therapeutic agents is IMM-124E. In some embodiments, one of the one or more additional therapeutic agents is KD-025. In some embodiments, one of the one or more additional therapeutic agents is linagliptin. In some embodiments, one of the one or more additional therapeutic agents is liraglutide. In some embodiments, one of the one or more additional therapeutic agents is mercaptamine. In some embodiments, one of the one or more additional therapeutic agents is MGL-3196. In some embodiments, one of the one or more additional therapeutic agents is obeticholic acid. In some embodiments, one of the one or more additional therapeutic agents is olesoxime. In some embodiments, one of the one or more additional therapeutic agents is peg-ilodecakin. In some embodiments, one of the one or more additional therapeutic agents is pioglitazone. In some embodiments, one of the one or more additional therapeutic agents is PX-102. In some embodiments, one of the one or more additional therapeutic agents is. In some embodiments, one of the one or more additional therapeutic agents is remogliflozin etabonate. In some embodiments, one of the one or more additional therapeutic agents is SHP-626. In some embodiments, one of the one or more additional therapeutic agents is solithromycin. In some embodiments, one of the one or more additional therapeutic agents is tipelukast. In some embodiments, one of the one or more additional therapeutic agents is TRX-318. In some embodiments, one of the one or more additional therapeutic agents is ursodeoxycholic acid. In some embodiments, one of the one or more additional therapeutic agents is and VBY-376.

In some embodiments, at least one of the one or more additional therapeutic agents is an anti-diabetic agent. In some embodiments, the anti-diabetic agent is an adenosine A₁ receptor agonist (e.g. adenosine, CCPA, CVT-3619, GR-190718), an adenosine A₂ receptor antagonist (istradefylline, SCH-58261), an aldose reductase inhibitor, an α-amylase inhibitor (e.g. tendamistat, treastatin, AL-3688), an α-glucosidase inhibitor (e.g. acarbose, camiglibose, diposine, emiglitate, miglitol, pradimicin-Q, sarbostatin, voglibose), an amylin analog (e.g. AC 164209 and pramlintide), an AMPK activator, a β3-adrenergic agonist (e.g. amibegron, AZ-40140, CL-316,243, KRP-204, L-742,791, L-796,568, LY-368,842, LY-377,604, mirabegron, Ro 40-2148, solabegron, SWR-0342SA), a β-ketoacyl-acyl carrier protein synthase inhibitor, a biguanide (e.g. metformin, buformin, phenformin), a carnitine palmitoyl transferase inhibitor, a DGAT-2 inhibitor, a DPP-4 inhibitor (e.g. alogliptin, anagliptin, dutogliptin, gemigliptin, linagliptin, omarigliptin, saxagliptin, sitagliptin, teneligliptin, trelagliptin, and vildagliptin), an ERN1 inhibitor, a fatty acid oxidation inhibitor, a fatty acid synthase (FAS) inhibitor, an FGF21 derivative, a fructose 1,6-diphosphatase inhibitor, a GLP1 agonist (e.g. albiglutide, dulaglutide, exenatide, liraglutide, lixisenatide, taspoglutide), a glucagon receptor modulator, a mixed glucagon receptor / GLP-1 agonist (e.g. MAR-701, ZP2929), a glucokinase inhibitor (e.g. TTP-399, TTP-355, TTP-547, AZD1656, ARRY403, MK-0599, TAK-329, AZD5658, and GKM-001), a glycogen phosphorylase inhibitor (e.g. GSK1362885), a GSK-3 inhibitor, a GPR119 agonist (e.g. MBX-2982, GSK1292263, APD597, PSN821), a GPBAR1 (TGR5) agonist (e.g. INT-777, XL-475), a GPR39 modulator, a GPR40 agonist (e.g. TAK-875), a GPR41 modulator, a GPR43 modulator, a GPR81 modulator, a GPR120 agonist, an HSL inhibitor, an IκB inhibitor, an ILI-beta modulator, insulin or an insulin analog (including, but not limited to, oral, inhaled or injectable formulations thereof), insulin-like growth factor (IGF-1) or an analog thereof, an insulin secretagogue, a JNK inhibitor (e.g. CC-359), a kappa opioid receptor modulator, LY3084077, a Kvl.3 inhibitor (e.g. ChTX, clofazmine, WIN-173173), a MAP4K4 inhibitor, an MC₁ or MC₄ agonist (e.g. afamelanotide, BMS-470539, bremelanotide, Melanotan II, PF-00446687, PL-6983, setmelanotide, and THIQ), a meglitinide (e.g. repaglinide, nateglinide, mitiglinide), a mineralocorticoid receptor inhibitor, a monoacylglycerol O-acyltransferase inhibitor, an NF-κB inhibitor, a nicotinic acid receptor (HM74A) activator, a PDE-10 inhibitor, a PDHK2 inhibitor, a PDHK4 inhibitor, a PKC (including PKC-alpha, PKC-beta, and PKC-gamma) inhibitor, a PPARα/γ dual agonist, a PTPlb inhibitor (e.g. trodusquemine), a retinol binding protein 4 inhibitor, a serine palmitoyl transferase inhibitor, an SGLT1 inhibitor (e.g. GSK1614235), a SIRT-1 inhibitor (e.g. resveratrol, GSK2245840, GSK184072), a somatostatin receptor inhibitor, a sulfonylurea (e.g. acetohexamide, chlorpropamide, diabinese, glibenclamide, glipizide, glyburide, blimipiride, gliclazide, glipentide, gliquidone, glisolamide, tolazamide, tolbutamide), a thiazolidinedione (e.g. ciglitazone, darglitazone, englitazone, lobeglitazone, MSDC-0602, netoglitazone, pioglitazone, rivoglitazone, rosiglitazone, and troglitazone), a TORC2 inhibitor, a urotensin II receptor agonist, a vasopressin agonist (e.g. DDAVP, WAY-141608), or a VPAC2 receptor agonist.

In some embodiments, at least one of the one or more additional therapeutic agents is an anti-antiobesity agent. In some embodiments, the anti-obesity agent is an apoB-MTP inhibitor (e.g. dirlotapide, JTT130, SLX4090, usistapide), a β3-adrenergic agonist (e.g. amibegron, AZ-40140, CL-316,243, KRP-204, L-742,791, L-796,568, LY-368,842, LY-377,604, mirabegron, Ro 40-2148, solabegron, SWR-0342SA), a bombesin receptor agonist, a BRS3 modulator, a CB1 receptor antagonist or inverse agonist, a CCK_{A} agonist, ciliary neurotrophic factor (CNTF) or analog thereof (e.g. axokine, NT-501), Contrave™ (buproprion/naltrexone), a dopamine receptor agonist (e.g. bromocriptine), an 11β-hydroxysteroid dehydrogenase (11β-HSD1) inhibitor, Empatic™ (pramlintide/metreleptin), a 5-HT_{2C} agonist (e.g. lorcaserin), a galanin antagonist, a ghrelin agonist or antagonist, a GLP1 agonist (e.g. albiglutide, dulaglutide, exenatide, liraglutide, lixisenatide, taspoglutide), a mixed glucagon receptor / GLP-1 agonist (e.g. MAR-701, ZP2929), an H3 antagonist or inverse agonist, a human agouti-related protein (AGRP) inhibitor, leptin or an analog thereof (e.g. metreleptin), a lipase inhibitor (e.g. tetrahydrolipstatin), an MC₁ or MC₄ agonist (e.g. afamelanotide, BMS-470539, bremelanotide, Melanotan II, PF-00446687, PL-6983, setmelanotide, and THIQ), a melanocyte-stimulating hormone or analog thereof, a MetAp2 inhibitor (e.g. ZGN-433), a monoamine reuptake inhibitor (e.g. buproprion, sibutramine, phentermine, tesofensine), a neuromedin U receptor agonist, an NPY antagonist (e.g. velneperit), an opioid receptor antagonist (e.g. naltrexone), an orexin receptor antagonist (e.g. almorexant, lemborexant, SB-334,867, SB-408,124, SB-649,868, suvorexant), oxyntomodulin or an analog thereof, PYY or an analog thereof (e.g. PYY₁₋₃₆, PYY₃₋₃₆), Qsymia™ (phentermine/topiramate), an RXR-alpha modulator, a stearoyl-CoA desaturase (SCD-1) inhibitor, or a sympathomimetic agent.

In some embodiments, at least one of the one or more additional therapeutic agents is a lipid lowering agent. In some embodiments, the lipid lowering agent is an acyl coenzyme A cholesterol acyl transferase (ACAT) inhibitor, a bile acid reabsorption inhibitor, a cholesterol ester transfer protein (CETP) inhibitor, a 5-LOX inhibitor (e.g. BAY X 1005), a FLAP inhibitor (e.g. AM-679), an HMG CoA synthase inhibitor, a lipoprotein synthesis inhibitor, a low-density lipoprotein receptor inducer, an LXR receptor modulator, a microsomal triglyceride transport inhibitor, niacin, a platelet aggregation inhibitor, a renin-angiotensin system inhibitor, a squalene epoxidase inhibitor, a squalene synthetase inhibitor, or a triglyceride synthesis inhibitor.

In some embodiments, at least one of the one or more additional therapeutic agents is an agent for treating a metabolic disorder. In some embodiments, the agent for treating a metabolic disorder is an ABC transporter activator, ACT-434964 (Actelion), an ANG-5 inhibitor, an angiotensin II antagonist (e.g. MC4262), CCX-872, DUR-928 (Durect), ESP41091, F-652 (Generon), an FGF21 agonist (e.g. BMS-986036), fomepizole (Raptor), an FXR agonist, FXR/TGR5 dual agonist (e.g. INT-767), a ghrelin antagonist (e.g. TZP-301), a glucosylceramide synthase inhibitor, a GPR17 modulator, a GPR119 agonist, IG-MD-014 (Indigene), IMM-124E (Immuron), a lysosome pathway modulator (e.g. CAT5000), a melanin-concentrating hormone receptor 1 antagonist (e.g. KI-1361-17), an MCL1 inhibitor (e.g. CMPX-1023), an mTORC1 inhibitor, an NaCT (e.g. SLC13A5) inhibitor, a NHE3 inhibitor (e.g. RDX-011, tenapanor), NP003 (Neuraltus), PBI-4050 (ProMetic), a proteostasis regulator (e.g. PTI-130, PTI-428, PTI-C1811), PS248288 (Pharmacopeia/Merck), PX-102 (Phenex), RG7410. RG7652, a ROCK inhibitor, SBC-104 (Synageva BioPharma), SPX-100 (Spherix), a stearoyl CoA desaturase inhibitor (e.g. CVT-12805), TRC150094 (Torrent), or ZYH7 (Zydus Cadila).

In some embodiments, at least one of the one or more additional therapeutic agents is an agent for treating steatosis. In some embodiments, the agent for treating steatosis is an adiponectin analog (e.g. PX 811013), aramchol (Galmed), an ASK1 inhibitor (e.g. GS4977, GS4997), AZD4076 (AstraZeneca), a bile acid sequestrant (e.g. obeticholic acid), BL-1060 (Galmed), BMS986171 (Bristol-Myers Squibb), a CCR5/CCR2 antagonist (e.g. cenicriviroc), cannabidiol, CER-209 (Cerenis), a cysteamine analog (e.g. RP-103, RP-104), DS102 (DS Biopharma), EGS21 (Enzo), elafibranor (Genfit), emricasan (Idun), ethyl eicosapentaenoic acid (Mochida), an FXR agonist, a GPBAR1 agonist (e.g. RDX009), GR-MD-02 (Galectin Therapeutics), leucine/sildenafil/metformin (NuSirt), LCQ908 (Novartis), LJN452 (Novartis), a LOXL2 inhibitor (e.g. simtuzumab), MAT-8800 (Matinas), MB-10866 (Metabasis), an miR-103/107 inhibitor (e.g. RG-125), MK-4074 (Merck & Co.), nalmefene (TaiwanJ), nivocasan (Gilead), NGM-282 (NGM Biopharmaceuticals), an omega-3 carboxylic acid or mixture of the same (e.g. Epanova™), PX-102 (Phenex), PX-104 (Phenex), remogliflozin etabonate (Kissei), saroglitazar (Zydus-Cadila), SAR-548304 (sanofi-aventis), tipelukast (Kyorin), ursodeoxycholic acid, VK2809 (Viking), or XL335 (Exelixis).

In some embodiments, at least one of the one or more additional therapeutic agents is an agent for treating inflammation. In some embodiments, the agent for treating inflammation reduces the differentiation or activation of Tₕ17 cells. In some embodiments, the agent for treating inflammation is a caspase inhibitor (e.g. emricasan), a TGF-β inhibitor, an IL-1β inhibitor, an IL-6 inhibitor, an IL-17 inhibitor, an IL-17a inhibitor, an IL-17F inhibitor, an IL-21 inhibitor, an IL-23 inhibitor (e.g. guselkumab), IMM-124E, a RORyt inhibitor (e.g. JTE-151) a RORα inhibitor, solithromycin (Cempra), or a vascular adhesion protein-1 inhibitor (e.g. PXS-4728A).

In some embodiments, at least one of the one or more additional therapeutic agents is an agent for treating fibrosis. In some embodiments, the agent for treating fibrosis is cenicriviroc (Tobira/Takeda), CNX-014/023/024/025 (Connexios), an endothelin antagonist (e.g. A192621, ambrisentan, atracentan, bosentan, BQ-123, BQ-788, macitentan, sitaxentan, tezosentan, zibotentan), etanercept, evitar (AdeTherapeutics), a fibroblast growth factor inhibitor, a galectin-3 inhibitor, imatinib, IVA337 (Inventiva), N-acetylcysteine, nintedanib, pirfenidone, RG6069 (Roche), SP20102 (Sarfez), tipelukast (Kyorin), or XOMA 089 (Xoma).

### Methods of Treatment

As described generally above, the present invention provides methods of treating, stabilizing or lessening the severity or progression of a non-alcoholic fatty liver disease comprising administering to a patient in need thereof an inhibitor of Acetyl-CoA carboxylase (ACC) in combination with one or more additional therapeutic agents.

In some embodiments, treatment is administered after one or more symptoms have developed. In other embodiments, treatment is administered in the absence of symptoms. For example, treatment is administered to a susceptible individual prior to the onset of symptoms (e.g., in light of a history of symptoms and/or in light of genetic or other susceptibility factors). Treatment is also continued after symptoms have resolved, for example to prevent, delay or lessen the severity of their recurrence.

In some embodiments, the non-alcoholic fatty liver disease is steatosis. In some embodiments, the non-alcoholic fatty liver disease is non-alcoholic steatohepatitis (NASH). In some embodiments, the non-alcoholic fatty liver disease is liver fibrosis caused by NASH. In some embodiments, the non-alcoholic fatty liver disease is liver cirrhosis caused by NASH. In some embodiments, the non-alcoholic fatty liver disease is hepatocellular carcinoma (HCC) caused by NASH.

### Combination Dosing

As described herein, provided methods comprise administration to a patient in need thereof an ACC inhibitor in combination with one or more additional therapeutic agents. As used herein, the term "in combination" with regard to administration of an ACC inhibitor and one or more therapeutic agents means that each of the ACC inhibitor and the one or more therapeutic agents can be administered to the patient in any order (i.e., simultaneously or sequentially) or together in a single composition, formulation, or unit dosage form.

It is understood that although the methods described herein may refer to formulations, doses and dosing regimens/schedules of ACC inhibitors, such formulations, doses and/or dosing regimens/schedules are equally applicable to any pharmaceutically acceptable salt of the ACC inhibitors. Accordingly, in some embodiments, a dose or dosing regimen for a pharmaceutically acceptable salt of an ACC inhibitor is selected from any of the doses or dosing regimens for ACC inhibitors as described herein.

It will be appreciated that the ACC inhibitor and the one or more additional therapeutic agents can be administered on the same day or on different days and in any order as according to an appropriate dosing protocol.

### Dosing of ACC inhibitor

In some embodiments, the present invention provides a method of treating, stabilizing or lessening the severity or progression of NAFLD comprising administering to a patient in need thereof one or more additional therapeutic agents in combination with a particular total daily dose of an ACC inhibitor, wherein the total daily dose of the ACC inhibitor is selected from about 10 mg, about 20 mg, about 30 mg, about 40 mg, about 50 mg, about 60 mg, about 70 mg, about 80 mg, about 90 mg, about 100 mg, about 150 mg, about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, about 2850 mg, about 2900 mg, about 2950 mg, or about 3000 mg.

In some embodiments, the present invention provides a method of treating, stabilizing or lessening the severity or progression of NAFLD comprising administering to a patient in need thereof on or more additional therapeutic agents in combination with a particular totoal daily dose of an ACC inhibitor, wherein the total daily dose of the ACC inhibitor is between about 10 mg to about 3000 mg, between about 10 mg to about 2000 mg, between about 10 mg to about 1000 mg, between about 20 mg to about 1000 mg, between about 30 mg to about 1000 mg, between about 30 mg to about 750 mg, between about 30 mg to about 500 mg, between about 30 mg to about 250 mg, between about 30 mg to about 100 mg, between about 50 mg to about 500 mg, and between about 50 mg to about 100 mg.

### Dosing of additional therapeutic agents

In some embodiments, the present invention provides methods for treating, stabilizing or lessening the severity or progression of NAFLD, comprising administering to a patient in need thereof a composition comprising an ACC inhibitor and one or more additional therapeutic agents, wherein each of the one or more additional therapeutic agents is administered in an amount of about 0.1 mg/day to about 1200 mg/day, about 1 mg/day to about 100 mg/day, about 10 mg/day to about 1200 mg/day, about 10 mg/day to about 100 mg/day, about 100 mg/day to about 1200 mg/day, about 400 mg/day to about 1200 mg/day, about 600 mg/day to about 1200 mg/day, about 400 mg/day to about 800 mg/day or about 600 mg/day to about 800 mg/day. In some embodiments, methods disclosed herein comprise the administration of 0.1 mg/day, 0.5 mg/day, 1 mg/day, 10 mg/day, 15 mg/day, 20 mg/day, 30 mg/day, 40 mg/day, 45 mg/day, 50 mg/day, 60 mg/day, 75 mg/day, 100 mg/day, 125 mg/day, 150 mg/day, 200 mg/day, 250 mg/day, 300 mg/day, 400 mg/day, 600 mg/day or 800 mg/day of a therapeutic agent to a patient in need thereof.

In some embodiments, the present invention provides methods for treating, stabilizing or lessening the severity or progression of NAFLD, comprising administering to a patient in need thereof a composition comprising an ACC inhibitor and one or more additional therapeutic agents, wherein the total daily dose of each therapeutic agents is selected from about 5 mg, about 10 mg, about 20 mg, about 25 mg, about 30mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, about 2850 mg, about 2900 mg, about 2950 mg, or about 3000 mg.

In some embodiments, the present invention provides a method of treating, stabilizing or lessening the severity or progression of NAFLD comprising administering to a patient in need thereof on or more additional therapeutic agents in combination with a particular totoal daily dose of an ACC inhibitor, wherein the total daily dose of each of the one or more additional therapeutic agents is independently between about 5 mg to about 3000 mg, between about 5 mg to about 1000 mg, between about 5 mg to about 500 mg, between about 5 mg to about 100 mg, 10 mg to about 3000 mg, between about 10 mg to about 2000 mg, between about 10 mg to about 1000 mg, between about 20 mg to about 1000 mg, between about 30 mg to about 1000 mg, between about 30 mg to about 750 mg, between about 30 mg to about 500 mg, between about 30 mg to about 250 mg, between about 30 mg to about 100 mg, between about 50 mg to about 500 mg, and between about 50 mg to about 100 mg.

### Unit Dosage Forms of ACC inhibitor

The ACC inhibitor is preferably formulated in unit dosage form for ease of administration and uniformity of dosage. It will be understood, however, that the total daily usage of the ACC inhibitor and compositions thereof, will be decided by the attending physician within the scope of sound medical judgment. The specific effective dose level for any particular patient or organism will depend upon a variety of factors including the disorder being treated and the severity of the disorder; the specific composition employed; the age, body weight, general health, sex and diet of the patient; the time of administration, route of administration, and rate of excretion of a given ACC inhibitor; the duration of the treatment; drugs used in combination or coincidental with the ACC inhibitor, and like factors well known in the medical arts. A person of ordinary skill will appreciate that the unit dosage forms described herein refer to an amount of an ACC inhibitor, which may be provided as the free acid or free base or as a pharmaceutically acceptable salt thereof.

In some embodiment, the present invention provides methods for treating, stabilizing or lessening the severity or progression of NAFLD, comprising administering to a patient in need thereof a composition comprising an ACC inhibitor and one or more additional therapeutic agents, wherein the ACC inhibitor is administered in unit dosage formulations that comprise between about 5 mg to about 1000 mg of ACC inhibitor. In certain embodiments, a unit dosage formulation of the present invention provides about 1 mg, 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 550 mg, about 600 mg, about 650 mg, about 700 mg, about 750 mg, about 800 mg, about 850 mg, about 900 mg, about 950 mg, or about 1000 mg of ACC inhibitor.

In some embodiments, the present invention provides methods for treating, stabilizing or lessening the severity or progression of NAFLD, comprising administering to a patient in need thereof a composition comprising an ACC inhibitor and one or more therapeutic agents, wherein the ACC inhibitor is administered in unit dosage formulations that comprise about 5 mg, 30 mg, or 150 mg of ACC inhibitor. In certain embodiments, a capsule formulation of the present invention provides about 25 mg, about 50 mg, about 75 mg, about 100 mg, about 125 mg, or about 150 mg of ACC inhibitor.

In certain embodiments, an ACC inhibitor is administered at dosage levels of about 0.01 mg/kg to about 50 mg/kg and preferably from about 1 mg/kg to about 25 mg/kg, of subject body weight per day, one or more times a day, to obtain the desired therapeutic effect.

### Unit Dosage Forms of additional therapeutic agents

In some embodiment, the present invention provides methods for treating, stabilizing or lessening the severity or progression of NAFLD, comprising administering to a patient in need thereof a composition comprising an ACC inhibitor and one or more additional therapeutic agents, wherein each of the one or more additional therapeutic agents is administered in unit dosage formulations that comprise between about 0.1 mg and about 2000 mg, about 1 mg and 200 mg, about 35 mg and about 1400 mg, about 125 mg and about 1000 mg, about 250 mg and about 1000 mg, or about 500 mg and about 1000 mg of a therapeutic agent.

In some embodiments, provided herein are unit dosage formulations comprising about 0.1 mg, 0.25 mg, 0.5 mg, 1 mg, 5 mg, 10 mg, 15 mg, 20 mg, 30 mg, 45 mg, 50 mg, 60 mg, 75 mg, 100 mg, 125 mg, 150 mg, 200 mg, 250 mg, 300 mg, 400 mg, 600 mg or 800 mg of a each additional therapeutic agent.

In some embodiments, provided herein are unit dosage formulations that comprise 0.1 mg, 0.25 mg, 0.5 mg, 1 mg, 2.5 mg, 5 mg, 10 mg, 15 mg, 20 mg, 30 mg, 35 mg, 50 mg, 70 mg, 100 mg, 125 mg, 140 mg, 175 mg, 200 mg, 250 mg, 280 mg, 350 mg, 500 mg, 560 mg, 700 mg, 750 mg, 1000 mg or 1400 mg of each additional therapeutic agent. In a particular embodiment, provided herein are unit dosage formulations that comprise about 5 mg, about 15 mg, about 20 mg, about 30 mg, about 45 mg, and about 50 mg of each additional therapeutic agent.

### Administration of ACC inhibitor

An ACC inhibitor, and compositions thereof according to methods of the present invention, are administered using any amount and any route of administration effective for treating or lessening the severity of NAFLD. The exact amount required will vary from subject to subject, depending on the species, age, and general condition of the subject, the severity of the disease, the particular agent, its mode of administration, and the like.

In some embodiments, provided methods comprise administering a pharmaceutically acceptable composition comprising an ACC inhibitor one, two, three, or four times a day.

In some embodiments, a pharmaceutically acceptable composition comprising an ACC inhibitor is administered once daily ("QD").

In some embodiments, a pharmaceutically acceptable composition comprising an ACC inhibitor is administered twice daily. In some embodiments, twice daily administration refers to a compound or composition that is administered "BID", or two equivalent doses administered at two different times in one day.

In some embodiments, a pharmaceutically acceptable composition comprising an ACC inhibitor is administered three times a day. In some embodiments, a pharmaceutically acceptable composition comprising an ACC inhibitor is administered "TID", or three equivalent doses administered at three different times in one day.

In some embodiments, a pharmaceutically acceptable composition comprising an ACC inhibitor is administered four times a day. In some embodiments, a pharmaceutically acceptable composition comprising an ACC inhibitor is administered "QID", or four equivalent doses administered at four different times in one day.

In some embodiments, an ACC inhibitor is administered to a patient under fasted conditions and the total daily dose is any of those contemplated above and herein.

In some embodiments, an ACC inhibitor is administered to a patient under fed conditions and the total daily dose is any of those contemplated above and herein.

In some embodiments, an ACC inhibitor is administered orally.

Pharmaceutically acceptable compositions of this invention can be administered to humans and other animals orally, rectally, parenterally, intracisternally, intravaginally, intraperitoneally, topically (as by powders, ointments, or drops), buccally, as an oral or nasal spray, or the like, depending on the severity of the disease or disorder being treated.

### Administration of additional therapeutic agents

In some embodiments, provided methods comprise administering a pharmaceutically acceptable composition comprising one or more additional therapeutic agents one, two, three, or four times a day.

In some embodiments, a pharmaceutically acceptable composition comprising one or more additional therapeutic agents is administered once daily ("QD").

In some embodiments, a pharmaceutically acceptable composition comprising one or more additional therapeutic agents is administered twice daily. In some embodiments, twice daily administration refers to a compound or composition that is administered "BID", or two equivalent doses administered at two different times in one day.

In some embodiments, a pharmaceutically acceptable composition comprising one or more additional therapeutic agents is administered three times a day. In some embodiments, a pharmaceutically acceptable composition comprising one or more additional therapeutic agents is administered "TID", or three equivalent doses administered at three different times in one day.

In some embodiments, a pharmaceutically acceptable composition comprising one or more additional therapeutic agents is administered four times a day. In some embodiments, a pharmaceutically acceptable composition comprising one or more additional therapeutic agents is administered "QID", or four equivalent doses administered at four different times in one day. In some embodiments, a pharmaceutically acceptable composition comprising one or more additional therapeutic agents is administered for a various number of days (for example 14, 21, 28) with a various number of days between treatment (0, 14, 21, 28).

In some embodiments, an additional therapeutic agent are administered to a patient under fasted conditions and the total daily dose is any of those contemplated above and herein.

In some embodiments, an additional therapeutic agent is administered to a patient under fed conditions and the total daily dose is any of those contemplated above and herein.

In some embodiments, an additional therapeutic agent is administered orally for reasons of convenience. In some embodiments, when administered orally, an additional therapeutic agent is administered with a meal and water. In another embodiment, an additional therapeutic agent is dispersed in water or juice (*e.g*., apple juice or orange juice) and administered orally as a suspension. In some embodiments, when administered orally, an additional therapeutic agent is administered in a fasted state.

A therapeutic agent can also be administered intradermally, intramuscularly, intraperitoneally, percutaneously, intravenously, subcutaneously, intranasally, epidurally, sublingually, intracerebrally, intravaginally, transdermally, rectally, mucosally, by inhalation, or topically to the ears, nose, eyes, or skin. The mode of administration is left to the discretion of the health-care practitioner, and can depend in-part upon the site of the medical condition.

### Pharmaceutically Acceptable Compositions of an ACC inhibitor and/or one or more additional therapeutic agents

In some embodiments, the present invention provides a pharmaceutically acceptable composition comprising an ACC inhibitor. In some embodiments, the present invention provides a pharmaceutically acceptable composition of a therapeutic agent. In some embodiments, a composition comprising an ACC inhibitor is separate from a composition comprising a therapeutic agent. In some embodiments, an ACC inhibitor and one or more additional therapeutic agents are present in the same composition.

Exemplary such pharmaceutically acceptable compositions are described further below and herein.

Liquid dosage forms for oral administration include, but are not limited to, pharmaceutically acceptable emulsions, microemulsions, solutions, suspensions, syrups and elixirs. In addition to an ACC inhibitor and/or one or more additional therapeutic agents, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of an ACC inhibitor, and/or the one or more additional therapeutic agents, it is often desirable to slow absorption from subcutaneous or intramuscular injection. This may be accomplished by the use of a liquid suspension of crystalline or amorphous material with poor water solubility. The rate of absorption then depends upon its rate of dissolution that, in turn, may depend upon crystal size and crystalline form. Alternatively, delayed absorption of parenterally administered ACC inhibitor and/or additional therapeutic agents, is accomplished by dissolving or suspending the compound in an oil vehicle. Injectable depot forms are made by forming microencapsule matrices of ACC inhibitor and/or additional therapeutic agents, in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polyethylene glycols and the like.

An ACC inhibitor and/or additional therapeutic agents, can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms an ACC inhibitor and/or additional therapeutic agents, may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of an ACC inhibitor and/or additional therapeutic agents, include ointments, pastes, creams, lotions, gels, powders, solutions, sprays, inhalants or patches. The active components are admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to one embodiment, the invention relates to a method of inhibiting *de novo* fatty acid synthesis in a biological sample comprising the step of contacting said biological sample with an ACC inhibitor and/or one or more additional therapeutic agents.

According to one embodiment, the invention relates to a method of increasing fatty acid oxidation in a biological sample comprising the step of contacting said biological sample with an ACC inhibitor and/or one or more additional therapeutic agents.

The term "biological sample", as used herein, includes, without limitation, cell cultures or extracts thereof; biopsied material obtained from a mammal or extracts thereof; and blood, saliva, urine, feces, semen, tears, or other body fluids or extracts thereof.

The invention will now be described with reference to the following clauses:
1. A method of treating, stabilizing, or lessening the severity or progression of a non-alcoholic fatty liver disease comprising administering to a patient in need thereof a composition comprising an ACC inhibitor.
2. The method of clause 1, wherein the ACC inhibitor is administered in combination with one or more additional therapeutic agents.
3. The method according to clauses 1 or 2, wherein the one or more additional therapeutic agents are independently selected from the group consisting of angiotensin II receptor antagonists, angiotensin converting enzyme (ACE) inhibitors, caspase inhibitors, cathepsin B inhibitors, CCR2 chemokine antagonists, CCR5 chemokine antagonists, chloride channel stimulators, cholesterol solubilizers, diacylglycerol O-acyltransferase 1 (DGAT1) inhibitors, dipeptidyl peptidase IV (DPPIV) inhibitors, farnesoid X receptor (FXR) agonists, galectin-3 inhibitors, glucagon-like peptide 1 (GLP1) agonists, glutathione precursors, hepatitis C virus NS3 protease inhibitors, HMG CoA reductase inhibitors, 11β-hydroxysteroid dehydrogenase (11β-HSD1) inhibitors, IL-1β antagonists, IL-6 antagonists, IL-10 agonists, IL-17 antagonists, ileal sodium bile acid cotransporter inhibitors, leptin analogs, 5-lipoxygenase inhibitors, LPL gene stimulators, lysyl oxidase homolog 2 (LOXL2) inhibitors, PDE3 inhibitors, PDE4 inhibitors, phospholipase C (PLC) inhibitors, PPARα agonists, PPAPγ agonists, PPARδ agonists, Rho associated protein kinase 2 (ROCK2) inhibitors, sodium glucose transporter-2 (SGLT2) inhibitors, stearoyl CoA desaturase-1 inhibitors, thyroid hormone receptor β agonists, tumor necrosis factor α (TNFα) ligand inhibitors, transglutaminase inhibitors, and transglutaminase inhibitor precursors.
4. The method according to any one of clauses 1-3, wherein the one or more additional therapeutic agents are independently selected from acetylsalicylic acid, alipogene tiparvovec, aramchol, atorvastatin, BLX-1002, cenicriviroc, cobiprostone, colesevelam, emricasan, enalapril, GFT-505, GR-MD-02, hydrochlorothiazide, icosapent ethyl ester (ethyl eicosapentaenoic acid), IMM-124E, KD-025, linagliptin, liraglutide, mercaptamine, MGL-3196, obeticholic acid, olesoxime, peg-ilodecakin, pioglitazone, PX-102, remogliflozin etabonate, SHP-626, solithromycin, tipelukast, TRX-318, ursodeoxycholic acid, and VBY-376.
5. The method according to any one of clauses 1-4, wherein the ACC inhibitor has the formula I: 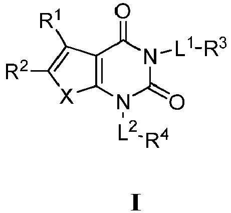 or a pharmaceutically acceptable salt thereof, wherein:
   X is -O-, -S-, or -NR-;
   R¹ is hydrogen or C₁₋₄ aliphatic, optionally substituted with one or more halogen, -OR, -SR, -N(R)₂, -N(R)C(O)R, -C(O)N(R)₂, -N(R)C(O)N(R)₂, -N(R)C(O)OR, -OC(O)N(R)₂, -N(R)SO₂R,-SO₂N(R)₂, -C(O)R, -C(O)OR, -OC(O)R, -S(O)R, or -SO₂R;
   R² is halogen, -R, -OR, -SR, -N(R)₂, -N(R)C(O)R, -C(O)N(R)₂, -N(R)C(O)N(R)₂,-N(R)C(O)OR, -OC(O)N(R)₂, -N(R)SO₂R, -SO₂N(R)₂, -C(O)R, -C(O)OR, -OC(O)R, -S(O)R, or -SO₂R, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
   or R¹ and R² are taken together to form an optionally substituted 4-7 membered partially unsaturated carbocyclo-, or heterocyclo-, benzo-, or 5-6 membered heteroarylo- fused ring;
   each R is independently hydrogen or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
   each of L¹ and L² is independently a covalent bond or an optionally substituted 1-6 membered straight or branched bivalent hydrocarbon chain; or a cyclopropylenyl, cyclobutylenyl, or oxetanyl group;
   R³ is hydrogen, halogen, -CN,-OR, -SR, -N(R)₂, -N(R)C(O)R, -C(O)N(R)₂, -C(O)N(R)S(O)₂R,-N(R)C(O)N(R)₂, -N(R)C(O)OR, -OC(O)N(R)₂, -N(R)SO₂R, -SO₂N(R)₂, -C(O)R,-C(O)OR, -OC(O)R, -S(O)R, -SO₂R, -B(OH)₂, or an optionally substituted ring selected from phenyl or 5-6 membered heteroaryl having 1-4 heteroatoms independently selected from nitrogen, oxygen, or sulfur; and
   R⁴ is hydrogen or an optionally substituted ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur.
6. The method according to any one of clauses 1-5, wherein the ACC inhibitor is: 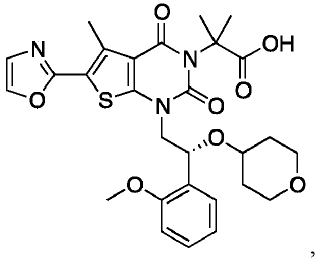 or a pharmaceutically acceptable salt thereof.
7. The method according to any one of clauses 1-5, wherein the ACC inhibitor is: 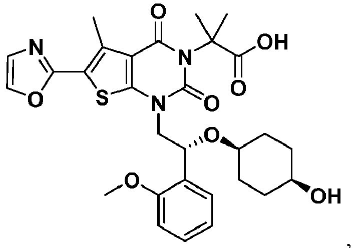 or a pharmaceutically acceptable salt thereof.
8. The method according to any one of clauses 1-5, wherein the ACC inhibitor has the general formula **II:** 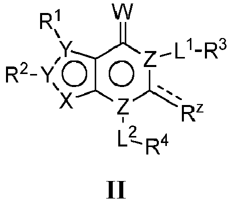 or a pharmaceutically acceptable salt thereof, wherein:
   W is oxygen or sulfur;
   X is O, S, N or NR;
   each Y is independently C, N, or O;
   each Z is independently C or N;
   R¹ is hydrogen or C₁₋₄ aliphatic, optionally substituted with one or more halogens, -OR, -SR,-N(R)₂, -N(R)C(O)R, -C(O)N(R)₂, -N(R)C(O)N(R)₂, -N(R)C(O)OR, -OC(O)N(R)₂,-N(R)SO₂R, -SO₂RN(R)₂, -C(O)R, -C(O)OR, -OC(O)R, -C(O)OR, -S(O)R, or -SO₂R;
   R² is halogen, -R, -OR, -SR, -N(R)₂, -N(R)C(O)R,-C(O)N(R)₂, -N(R)C(O)N(R)₂, -N(R)C(O)OR, -OC(O)N(R)₂, -N(R)SO₂R,-SO₂N(R)₂, -C(O)R, -C(O)OR, -OC(O)R, -S(O)R, -SO₂R, -B(OR)₂, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; wherein R² is not optionally substituted benzyl; or
   R¹ and R² are taken together to form an optionally substituted 4-7 membered partially unsaturated carbocyclo-, or heterocyclo-, benzo-, or 5-6 membered heteroarylo- fused ring;
   each R is independently hydrogen, deuterium, or an optionally substituted group selected from C_{1- 6} aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
   L¹ is a covalent bond or a 1-6 membered straight or branched bivalent hydrocarbon chain optionally substituted with R⁵ and R^{5'};
   L² is a covalent bond or a 1-6 membered straight or branched bivalent hydrocarbon chain optionally substituted with R⁷ and R^{7'};
   R³ is halogen, -CN, -OR, -SR, -N(R)₂, -N(R)C(O)R, -C(O)RN(R)₂, -C(O)N(R)S(O)₂R,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
   R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from
   nitrogen, oxygen, or sulfur; wherein said ring is optionally substituted with n instances of R⁸;
   each of R⁵ and R⁵' is independently -R, -OR, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, or -SO₂R; or R⁵ and R⁵ are taken together to form a cyclopropylenyl, cyclobutylenyl, or oxetanyl group;
   each of R⁷ and R^{7'} is independently, -R, -OR⁶, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
   R⁶ is -R, -C(O)N(R)₂, or -C(O)R;
   each R⁸ is independently selected from halogen, -R, -OR, -SR, -N(R)₂ or deuterium;
   R^{z} is selected from hydrogen, halogen, methyl, -CN, =O, and =S; and
   n is 0-5.
9. The method according to any one of clauses 1-5, wherein the ACC inhibitor has the general formula **III:** 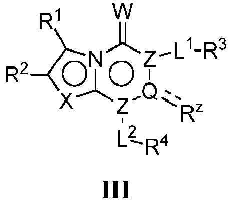 or a pharmaceutically acceptable salt thereof, wherein:
   W is oxygen or sulfur;
   Q is C or N; wherein if Q is N, then R^{Z} is absent;
   X is -O-, -S-, or -NR-;
   each Z is independently C or N; provided that both Z are not N;
   R¹ is hydrogen or C₁₋₄ aliphatic, optionally substituted with one or more halogens, -OR, -SR,-N(R)₂, -N(R)C(O)R, -C(O)N(R)₂, -N(R)C(O)N(R)₂, -N(R)C(O)OR, -OC(O)N(R)₂,-N(R)SO₂R, -SO₂RN(R)₂, -C(O)R, -C(O)OR, -OC(O)R, -C(O)OR, -S(O)R, or -SO₂R;
   R² is halogen, -R, -OR, -SR, -N(R)₂, -N(R)C(O)R, -C(O)N(R)₂, -N(R)C(O)N(R)₂,-N(R)C(O)OR, -OC(O)N(R)₂, -N(R)SO₂R, -SO₂N(R)₂, -C(O)R, -C(O)OR, -OC(O)R,-S(O)R, -SO₂R, -B(OR)₂, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; or
   R¹ and R² are taken together to form an optionally substituted 4-7 membered partially unsaturated carbocyclo-, or heterocyclo-, benzo-, or 5-6 membered heteroarylo- fused ring;
   each R is independently hydrogen, deuterium, or an optionally substituted group selected from C_{1- 6} aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
   L¹ is a covalent bond or a 1-6 membered straight or branched bivalent hydrocarbon chain optionally substituted with R⁵ and R^{5'};
   L² is a covalent bond or a 1-6 membered straight or branched bivalent hydrocarbon chain optionally substituted with R⁷ and R^{7'};
   R³ is halogen, -CN, -OR, -SR, -N(R)₂, -N(R)C(O)R, -C(O)RN(R)₂, -C(O)N(R)S(O)₂R,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
   R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
   each of R⁵ and R⁵' is independently -R, -OR, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, or -SO₂R; or R⁵ and R^{5'} are taken together to form a cyclopropylenyl, cyclobutylenyl, or oxetanyl group; and
   each of R⁷ and R^{7'} is independently, -R, -OR⁶, -SR, -N(R)₂, -N(R)C(O)R, -C(O)N(R)₂,-N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
   R⁶ is -R, -C(O)N(R)₂, or -C(O)R;
   each R⁸ is independently selected from halogen, -R, -OR, -SR, -N(R)₂ or deuterium;
   R^{Z} is selected from hydrogen, halogen, methyl, -CN, =O, and =S; and
   n is 0-5.
10. The method according to any one of clauses 1-5, wherein the ACC inhibitor has the general formula **IV:** 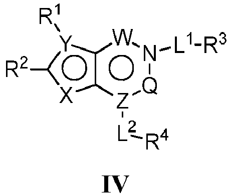 or a pharmaceutically acceptable salt thereof, wherein:
   W is -C(O)-, -C(S)-, or -S(O)₂-;
   Q is -C(O)-, -C(S)-, -S(O)₂-, or N;
   X is -O-, -S-, -NR-, or N;
   Y is C or N;
   Z is C or N;
   R¹ is hydrogen or C₁₋₄ aliphatic, optionally substituted with one or more halogens, -OR, -SR,-N(R)₂, -N(R)C(O)R, -C(O)N(R)₂, -N(R)C(O)N(R)₂, -N(R)C(O)OR, -OC(O)N(R)₂,-N(R)SO₂R, -SO₂RN(R)₂, -C(O)R, -C(O)OR, -OC(O)R, -C(O)OR, -S(O)R, or -SO₂R;
   R² is halogen, -R, -OR, -SR, -N(R)₂, -N(R)C(O)R, - C(O)N(R)₂, -N(R)C(O)N(R)₂, -N(R)C(O)OR, -OC(O)N(R)₂, -N(R)SO₂R, - SO₂N(R)₂, -C(O)R, -C(O)OR, -OC(O)R, -S(O)R, or -SO₂R, -B(OR)₂, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; wherein R² is not optionally substituted benzyl; or
   R¹ and R² are taken together to form an optionally substituted 4-7 membered partially unsaturated carbocyclo-, or heterocyclo-, benzo-, or 5-6 membered heteroarylo- fused ring;
   each R is independently hydrogen, deuterium, or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
   L¹ is a covalent bond or a 1-6 membered straight or branched bivalent hydrocarbon chain optionally substituted with R⁵ and R^{5'};
   L² is a covalent bond or a 1-6 membered straight or branched bivalent hydrocarbon chain optionally substituted with R⁷ and R^{7'};
   R³ is halogen, -CN, -OR, -SR, -N(R)₂, -N(R)C(O)R, -C(O)RN(R)₂, -C(O)N(R)S(O)₂R, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
   R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
   each of R⁵ and R^{5'} is independently -R, -OR, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, or -SO₂R; or R⁵ and R^{5'} are taken together to form a cyclopropylenyl, cyclobutylenyl, or oxetanyl group;
   each of R⁷ and R^{7'} is independently, -R, -OR⁶, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, -SO₂R, or -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
   R⁶ is -R, -C(O)N(R)₂, or -C(O)R;
   each R⁸ is independently selected from halogen, -R, -OR, -SR, -N(R)₂ or deuterium; and n is 0-5.
11. The method according to any one of clauses 1-5, wherein the ACC inhibitor has the formula V: 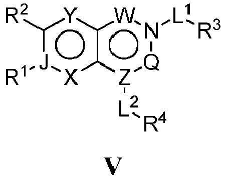 or a pharmaceutically acceptable salt thereof, wherein:
   W is -C(R^{z})-, -C(O)-, or -C(S)-;
   Q is -C(R^{z})-, -C(O)-, or -C(S)-;
   J is C or N; provided that when J is N, R¹ is absent;
   X is CH or N;
   Y is CH or N;
   Z is C or N;
   R¹ is hydrogen or C₁₋₄ aliphatic, optionally substituted with one or more halogens, -OR, -SR, - N(R)₂, -N(R)C(O)R, -C(O)N(R)₂, -N(R)C(O)N(R)₂, -N(R)C(O)OR, -OC(O)N(R)₂, - N(R)SO₂R, -SO₂RN(R)₂, -C(O)R, -C(O)OR, -OC(O)R, -C(O)OR, -S(O)R, or -SO₂R;
   R² is halogen, -R, -OR, -SR, -N(R)₂, -N(R)C(O)R, -C(O)N(R)₂, -N(R)C(O)N(R)₂, - N(R)C(O)OR, -OC(O)N(R)₂, -N(R)SO₂R, -SO₂N(R)₂, -C(O)R, -C(O)OR, -OC(O)R, - S(O)R, -B(OR)₂, -SO₂R or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; or
   R¹ and R² are taken together to form an optionally substituted 4-7 membered partially unsaturated carbocyclo-, or heterocyclo-, benzo-, or 5-6 membered heteroarylo- fused ring;
   each R is independently hydrogen, deuterium, or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
   L¹ is a covalent bond or a 1-6 membered straight or branched bivalent hydrocarbon chain optionally substituted with R⁵ and R^{5'};
   L² is a covalent bond or a 1-6 membered straight or branched bivalent hydrocarbon chain optionally substituted with R⁷ and R^{7'};
   R³ is hydrogen, halogen, -CN, -OR, -SR, -N(R)₂, -N(R)C(O)R, -C(O)RN(R)₂, -C(O)N(R)S(O)₂R, -N(R)C(O)N(R)₂, -N(R)C(O)OR, -OC(O)N(R)₂, -N(R)SO₂R, -SO₂N(R)₂, -C(O)R, -C(O)OR, -OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
   R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
   each of R⁵ and R^{5'} is independently -R, -OR, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, or -SO₂R; or R⁵ and R^{5'} are taken together to form a cyclopropylenyl, cyclobutylenyl, or oxetanyl group;
   each of R⁷ and R^{7'} is independently, -R, -OR⁶, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, -SO₂R, or -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
   R⁶ is -R, -C(O)N(R)₂, or -C(O)R;
   each R⁸ is independently selected from halogen, -R, -OR, -SR, -N(R)₂ or deuterium;
   each R^{z} is independently selected from the group consisting of hydrogen, halogen, C₁-C₃ alkyl, and -CN; and
   n is 0-5.
12. The method according to any one of clauses 2-11, wherein the ACC inhibitor and the one or more additional therapeutic agents are administered simultaneously.
13. The method according to any one of clauses 2-11, wherein the ACC inhibitor and the one or more additional therapeutic agents are administered sequentially.
14. The method according to any one of clauses 2-13, wherein the non-alcoholic fatty liver disease is steatosis.
15. The method according to any one of clauses 1-13, wherein the non-alcoholic fatty liver disease is non-alcoholic steatohepatitis (NASH).
16. The method according to any one of clauses 1-13, wherein the non-alcoholic fatty liver disease is liver fibrosis caused by NASH.
17. The method according to any one of clauses 1-13, wherein the non-alcoholic fatty liver disease is liver cirrhosis caused by NASH.
18. The method according to any one of clauses 1-13, wherein the non-alcoholic fatty liver disease is hepatocellular carcinoma (HCC) caused by NASH.
19. A system for treating, stabilizing or lessening the severity of a non-alcoholic fatty liver disease, the system comprising an ACC inhibitor, in combination with one or more additional therapeutic agents.
20. The system according to clause 19, wherein at least one of the one or more additional therapeutic agents is an anti-diabetic agent.
21. The system according to clause 20, wherein the anti-diabetic agent is an adenosine A₁ receptor agonist (e.g. adenosine, CCPA, CVT-3619, GR-190718), an adenosine A₂ receptor antagonist (e.g. istradefylline, SCH-58261), an aldose reductase inhibitor, an α-amylase inhibitor (e.g. tendamistat, treastatin, AL-3688), an α-glucosidase inhibitor (e.g. acarbose, camiglibose, diposine, emiglitate, miglitol, pradimicin-Q, sarbostatin, voglibose), an amylin analog (e.g. AC164209 and pramlintide), an AMPK activator, a β3-adrenergic agonist (e.g. amibegron, AZ-40140, CL-316,243, KRP-204, L-742,791, L-796,568, LY-368,842, LY-377,604, mirabegron, Ro 40-2148, solabegron, SWR-0342SA), a β-ketoacyl-acyl carrier protein synthase inhibitor, a biguanide (e.g. metformin, buformin, phenformin), a carnitine palmitoyl transferase inhibitor, a DGAT-2 inhibitor, a DPP-4 inhibitor (e.g. alogliptin, anagliptin, dutogliptin, gemigliptin, linagliptin, omarigliptin, saxagliptin, sitagliptin, teneligliptin, trelagliptin, and vildagliptin), an ERN1 inhibitor, a fatty acid oxidation inhibitor, a fatty acid synthase (FAS) inhibitor, an FGF21 derivative, a fructose 1,6-diphosphatase inhibitor, a GLP1 agonist (e.g. albiglutide, dulaglutide, exenatide, liraglutide, lixisenatide, taspoglutide), a glucagon receptor modulator, a mixed glucagon receptor / GLP-1 agonist (e.g. MAR-701, ZP2929), a glucokinase inhibitor (e.g. TTP-399, TTP-355, TTP-547, AZD1656, ARRY403, MK-0599, TAK-329, AZD5658, and GKM-001), a glycogen phosphorylase inhibitor (e.g. GSK1362885), a GSK-3 inhibitor, a GPR119 agonist (e.g. MBX-2982, GSK1292263, APD597, PSN821), a GPBAR1 (TGR5) agonist (e.g. INT-777, XL-475), a GPR39 modulator, a GPR40 agonist (e.g. TAK-875), a GPR41 modulator, a GPR43 modulator, a GPR81 modulator, a GPR120 agonist, an HSL inhibitor, an IκB inhibitor, an ILI-beta modulator, insulin or an insulin analog (including, but not limited to, oral, inhaled or injectable formulations thereof), insulin-like growth factor (IGF-1) or an analog thereof, an insulin secretagogue, a JNK inhibitor (e.g. CC-359), a kappa opioid receptor modulator, LY3084077, a Kvl.3 inhibitor (e.g. ChTX, clofazmine, WIN-173173), a MAP4K4 inhibitor, an MC₁ or MC₄ agonist (e.g. afamelanotide, BMS-470539, bremelanotide, Melanotan II, PF-00446687, PL-6983, setmelanotide, and THIQ), a meglitinide (e.g. repaglinide, nateglinide, mitiglinide), a mineralocorticoid receptor inhibitor, a monoacylglycerol O-acyltransferase inhibitor, an NF-κB inhibitor, a nicotinic acid receptor (HM74A) activator, a PDE-10 inhibitor, a PDHK2 inhibitor, a PDHK4 inhibitor, a PKC (including PKC-alpha, PKC-beta, and PKC-gamma) inhibitor, a PPARα/γ dual agonist, a PTPlb inhibitor (e.g. trodusquemine), a retinol binding protein 4 inhibitor, a serine palmitoyl transferase inhibitor, an SGLT1 inhibitor (e.g. GSK1614235), a SIRT-1 inhibitor (e.g. resveratrol, GSK2245840, GSK184072), a somatostatin receptor inhibitor, a sulfonylurea (e.g. acetohexamide, chlorpropamide, diabinese, glibenclamide, glipizide, glyburide, blimipiride, gliclazide, glipentide, gliquidone, glisolamide, tolazamide, tolbutamide), a thiazolidinedione (e.g. ciglitazone, darglitazone, englitazone, lobeglitazone, MSDC-0602, netoglitazone, pioglitazone, rivoglitazone, rosiglitazone, and troglitazone), a TORC2 inhibitor, a urotensin II receptor agonist, a vasopressin agonist (e.g. DDAVP, WAY-141608), or a VPAC2 receptor agonist.
22. The system according to clause 19, wherein at least one of the one or more additional therapeutic agents is an anti-obesity agent.
23. The system according to clause 22, wherein the anti-obesity agent is an apoB-MTP inhibitor (e.g. dirlotapide, JTT130, SLX4090, usistapide), a β3-adrenergic agonist (e.g. amibegron, AZ-40140, CL-316,243, KRP-204, L-742,791, L-796,568, LY-368,842, LY-377,604, mirabegron, Ro 40-2148, solabegron, SWR-0342SA), a bombesin receptor agonist, a BRS3 modulator, a CB1 receptor antagonist or inverse agonist, a CCK_{A} agonist, ciliary neurotrophic factor (CNTF) or analog thereof (e.g. axokine, NT-501), buproprion/naltrexone, a dopamine receptor agonist (e.g. bromocriptine), an 11β-hydroxysteroid dehydrogenase (11β-HSD1) inhibitor, pramlintide/metreleptin, a 5-HT_{2C} agonist (e.g. lorcaserin), a galanin antagonist, a ghrelin agonist or antagonist, a GLP1 agonist (e.g. albiglutide, dulaglutide, exenatide, liraglutide, lixisenatide, taspoglutide), a mixed glucagon receptor / GLP-1 agonist (e.g. MAR-701, ZP2929), an H3 antagonist or inverse agonist, a human agouti-related protein (AGRP) inhibitor, leptin or an analog thereof (e.g. metreleptin), a lipase inhibitor (e.g. tetrahydrolipstatin), an MC₁ or MC₄ agonist (e.g. afamelanotide, BMS-470539, bremelanotide, Melanotan II, PF-00446687, PL-6983, setmelanotide, and THIQ), a melanocyte-stimulating hormone or analog thereof, a MetAp2 inhibitor (e.g. ZGN-433), a monoamine reuptake inhibitor (e.g. buproprion, sibutramine, phentermine, tesofensine), a neuromedin U receptor agonist, an NPY antagonist (e.g. velneperit), an opioid receptor antagonist (e.g. naltrexone), an orexin receptor antagonist (e.g. almorexant, lemborexant, SB-334,867, SB-408,124, SB-649,868, suvorexant), oxyntomodulin or an analog thereof, PYY or an analog thereof (e.g. PYY₁₋₃₆, PYY₃₋₃₆), phentermine/topiramate, an RXR-alpha modulator, a stearoyl-CoA desaturase (SCD-1) inhibitor, or a sympathomimetic agent.
24. The system according to clause 19, wherein at least one of the one or more additional therapeutic agents is an agent for treating a metabolic disorder.
25. The system according to clause 24, wherein the agent for treating a metabolic disorder is is an ABC transporter activator, ACT-434964 (Actelion), an ANG-5 inhibitor, an angiotensin II antagonist (e.g. MC4262), CCX-872, DUR-928 (Durect), ESP41091, F-652 (Generon), an FGF21 agonist (e.g. BMS-986036), fomepizole (Raptor), an FXR agonist, dual FXR/TGR5 agonist (e.g. INT-767), a ghrelin antagonist (e.g. TZP-301), a glucosylceramide synthase inhibitor, a GPR17 modulator, a GPR119 agonist, IG-MD-014 (Indigene), IMM-124E (Immuron), a lysosome pathway modulator (e.g. CAT5000), a melanin-concentrating hormone receptor 1 antagonist (e.g. KI-1361-17), an MCL1 inhibitor (e.g. CMPX-1023), an mTORC1 inhibitor, an NaCT (e.g. SLC13A5) inhibitor, a NHE3 inhibitor (e.g. RDX-011, tenapanor), NP003 (Neuraltus), PBI-4050 (ProMetic), a proteostasis regulator (e.g. PTI-130, PTI-428, PTI-C1811), PS248288 (Pharmacopeia/Merck), PX-102 (Phenex), RG7410. RG7652, a ROCK inhibitor, SBC-104 (Synageva BioPharma), SPX-100 (Spherix), a stearoyl CoA desaturase inhibitor (e.g. CVT-12805), TRC150094 (Torrent), or ZYH7 (Zydus Cadila).
26. The system according to clause 19, wherein at least one of the one or more additional therapeutic agents is an agent for treating steatosis.
27. The system according to clause 26, wherein the agent for treating steatosis is an adiponectin analog (e.g. PX 811013), aramchol (Galmed), an ASK1 inhibitor (e.g. GS4977, GS4997), AZD4076 (AstraZeneca), a bile acid sequestrant (e.g. obeticholic acid), BL-1060 (Galmed), BMS986171 (Bristol-Myers Squibb), a CCR5/CCR2 antagonist (e.g. cenicriviroc), cannabidiol, CER-209 (Cerenis), cysteamine analog (e.g. RP-103, RP-104), DS102 (DS Biopharma), EGS21 (Enzo), elafibranor (Genfit), emricasan (Idun), ethyl eicosapentaenoic acid (Mochida), an FXR agonist, a GPBAR1 agonist (e.g. RDX009), GR-MD-02 (Galectin Therapeutics), leucine/sildenafil/metformin (NuSirt), LCQ908 (Novartis), LJN452 (Novartis), a LOXL2 inhibitor (e.g. simtuzumab), MAT-8800 (Matinas), MB-10866 (Metabasis), an miR-103/107 inhibitor (e.g. RG-125), MK-4074 (Merck & Co.), nalmefene (TaiwanJ), nivocasan (Gilead), NGM-282 (NGM Biopharmaceuticals), an omega-3 carboxylic acid or mixture of the same, PX-102 (Phenex), PX-104 (Phenex), remogliflozin etabonate (Kissei), saroglitazar (Zydus-Cadila), SAR-548304 (sanofi-aventis), tipelukast (Kyorin), ursodeoxycholic acid, VK2809 (Viking), or XL335 (Exelixis).
28. The system according to clause 19, wherein at least one of the one or more additional therapeutic agents is an agent for treating inflammation.
29. The system according to clause 28, wherein the agent for treating inflammation reduces the differentiation or activation of Tₕ17 cells.
30. The system according to clause 28, wherein the agent for treating inflammation is a caspase inhibitor (e.g. emricasan), a TGF-β inhibitor, an IL-1β inhibitor, an IL-6 inhibitor, an IL-17 inhibitor, an IL-17a inhibitor, an IL-17F inhibitor, an IL-21 inhibitor, an IL-23 inhibitor (e.g. guselkumab), IMM-124E (Immuron), a RORyt inhibitor (e.g. JTE-151), a RORα inhibitor, solithromycin (Cempra), or a vascular adhesion protein-1 inhibitor (e.g. PXS-4728A).
31. The system according to clause 19, wherein at least one of the one or more additional therapeutic agents is an agent for treating fibrosis.
32. The system according to clause 31, wherein the agent for treating fibrosis is fibrosis is CNX-014/023/024/025 (Connexios), evitar (AdeTherapeutics), a galectin-3 inhibitor, IVA337 (Inventiva), pirfenidone, RG6069 (Roche), SP20102 (Sarfez), or XOMA 089 (Xoma).
33. The system according to clause 19, wherein the one or more additional therapeutic agents are independently selected from the group consisting of angiotensin II receptor antagonists, angiotensin converting enzyme (ACE) inhibitors, caspase inhibitors, cathepsin B inhibitors, CCR2 chemokine antagonists, CCR5 chemokine antagonists, chloride channel stimulators, cholesterol solubilizers, diacylglycerol O-acyltransferase 1 (DGAT1) inhibitors, dipeptidyl peptidase IV (DPPIV) inhibitors, farnesoid X receptor (FXR) agonists, galectin-3 inhibitors, glucagon-like peptide 1 (GLP1) agonists, glutathione precursors, hepatitis C virus NS3 protease inhibitors, HMG CoA reductase inhibitors, 11β-hydroxysteroid dehydrogenase (11β-HSD1) inhibitors, IL-1β antagonists, IL-6 antagonists, IL-10 agonists, IL-17 antagonists, ileal sodium bile acid cotransporter inhibitors, leptin analogs, 5-lipoxygenase inhibitors, LPL gene stimulators, lysyl oxidase homolog 2 (LOXL2) inhibitors, PDE3 inhibitors, PDE4 inhibitors, phospholipase C (PLC) inhibitors, PPARα agonists, PPAPγ agonists, PPAPδ agonists, Rho associated protein kinase 2 (ROCK2) inhibitors, sodium glucose transporter-2 (SGLT2) inhibitors, stearoyl CoA desaturase-1 inhibitors, thyroid hormone receptor β agonists, tumor necrosis factor α (TNFα) ligand inhibitors, transglutaminase inhibitors, transglutaminase inhibitor precursors, PTPlb inhibitors, and ASK1 inhibitors.
34. The system according to clause 19, wherein the one or more additional therapeutic agents are independently selected from acetylsalicylic acid, alipogene tiparvovec, aramchol, atorvastatin, BLX-1002, cenicriviroc, cobiprostone, colesevelam, emricasan, enalapril, GFT-505, GR-MD-02, hydrochlorothiazide, icosapent ethyl ester (ethyl eicosapentaenoic acid), IMM-124E, KD-025, linagliptin, liraglutide, mercaptamine, MGL-3196, obeticholic acid, olesoxime, peg-ilodecakin, pioglitazone, PX-102, remogliflozin etabonate, SHP-626, solithromycin, tipelukast, TRX-318, ursodeoxycholic acid, and VBY-376.
35. The system according to any one of clauses 19-34, wherein the ACC inhibitor is: 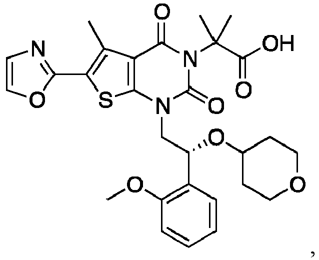 or a pharmaceutically acceptable salt thereof.
36. The system according to any one of clauses 19-35, wherein the ACC inhibitor is: 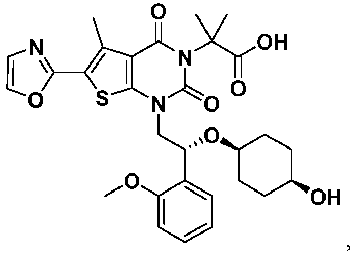 or a pharmaceutically acceptable salt thereof.
37. A composition comprising an ACC inhibitor, one or more additional therapeutic agents, and a pharmaceutically acceptable carrier, adjuvant, or vehicle.

## Claims

1. A composition comprising an ACC inhibitor for use in a method of treating, stabilizing, or lessening the severity or progression of a non-alcoholic fatty liver disease comprising administering to a patient in need thereof the composition comprising the ACC inhibitor,
optionally wherein the ACC inhibitor is administered in combination with one or more additional therapeutic agents.

2. The composition for the use according to claim 1, wherein the ACC inhibitor is administered in combination with one or more additional therapeutic agents; and
wherein the one or more additional therapeutic agents are independently selected from the group consisting of angiotensin II receptor antagonists, angiotensin converting enzyme (ACE) inhibitors, caspase inhibitors, cathepsin B inhibitors, CCR2 chemokine antagonists, CCR5 chemokine antagonists, chloride channel stimulators, cholesterol solubilizers, diacylglycerol O-acyltransferase 1 (DGAT1) inhibitors, dipeptidyl peptidase IV (DPPIV) inhibitors, farnesoid X receptor (FXR) agonists, galectin-3 inhibitors, glucagon-like peptide 1 (GLP1) agonists, glutathione precursors, hepatitis C virus NS3 protease inhibitors, HMG CoA reductase inhibitors, 11β-hydroxysteroid dehydrogenase (11β-HSD1) inhibitors, IL-1β antagonists, IL-6 antagonists, IL-10 agonists, IL-17 antagonists, ileal sodium bile acid cotransporter inhibitors, leptin analogs, 5-lipoxygenase inhibitors, LPL gene stimulators, lysyl oxidase homolog 2 (LOXL2) inhibitors, PDE3 inhibitors, PDE4 inhibitors, phospholipase C (PLC) inhibitors, PPARα agonists, PPARγ agonists, PPARδ agonists, Rho associated protein kinase 2 (ROCK2) inhibitors, sodium glucose transporter-2 (SGLT2) inhibitors, stearoyl CoA desaturase-1 inhibitors, thyroid hormone receptor β agonists, tumor necrosis factor α (TNFα) ligand inhibitors, transglutaminase inhibitors, and transglutaminase inhibitor precursors, or
wherein the one or more additional therapeutic agents are independently selected from acetylsalicylic acid, alipogene tiparvovec, aramchol, atorvastatin, BLX-1002, cenicriviroc, cobiprostone, colesevelam, emricasan, enalapril, GFT-505, GR-MD-02, hydrochlorothiazide, icosapent ethyl ester (ethyl eicosapentaenoic acid), IMM-124E, KD-025, linagliptin, liraglutide, mercaptamine, MGL-3196, obeticholic acid, olesoxime, peg-ilodecakin, pioglitazone, PX-102, remogliflozin etabonate, SHP-626, solithromycin, tipelukast, TRX-318, ursodeoxycholic acid, and VBY-376.

3. The composition for the use according to claim 1 or 2, wherein the ACC inhibitor has the formula I: 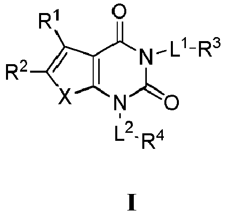 or a pharmaceutically acceptable salt thereof, wherein:
X is -O-, -S-, or -NR-;
R¹ is hydrogen or C₁₋₄aliphatic, optionally substituted with one or more halogen, -OR, -SR, -N(R)₂, -N(R)C(O)R, -C(O)N(R)₂, -N(R)C(O)N(R)₂, -N(R)C(O)OR, -OC(O)N(R)₂, -N(R)SO₂R, - SO₂N(R)₂, -C(O)R, -C(O)OR, -OC(O)R, -S(O)R, or -SO₂R;
R² is halogen, -R, -OR, -SR, -N(R)₂, -N(R)C(O)R, -C(O)N(R)₂, -N(R)C(O)N(R)₂, - N(R)C(O)OR, -OC(O)N(R)₂, -N(R)SO₂R, -SO₂N(R)₂, -C(O)R, -C(O)OR, -OC(O)R, -S(O)R, or -SO₂R, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
or R¹ and R² are taken together to form an optionally substituted 4-7 membered partially unsaturated carbocyclo-, or heterocyclo-, benzo-, or 5-6 membered heteroarylo- fused ring;
each R is independently hydrogen or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
each of L¹ and L² is independently a covalent bond or an optionally substituted 1-6 membered straight or branched bivalent hydrocarbon chain; or a cyclopropylenyl, cyclobutylenyl, or oxetanyl group;
R³ is hydrogen, halogen, -CN,-OR, -SR, -N(R)₂, -N(R)C(O)R, -C(O)N(R)₂, -C(O)N(R)S(O)₂R, - N(R)C(O)N(R)₂, -N(R)C(O)OR, -OC(O)N(R)₂, -N(R)SO₂R, -SO₂N(R)₂, -C(O)R, - C(O)OR, -OC(O)R, -S(O)R, -SO₂R, -B(OH)₂, or an optionally substituted ring selected from phenyl or 5-6 membered heteroaryl having 1-4 heteroatoms independently selected from nitrogen, oxygen, or sulfur; and
R⁴ is hydrogen or an optionally substituted ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur.

4. The composition for the use according to any one of claims 1-3, wherein the ACC inhibitor is: 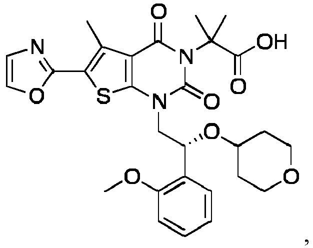 or a pharmaceutically acceptable salt thereof, or 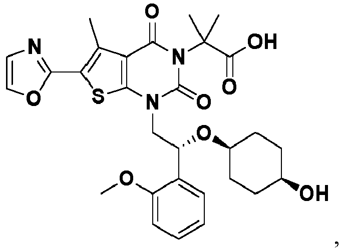 or a pharmaceutically acceptable salt thereof.

5. The composition for the use according to any one of claims 1-3, wherein the ACC inhibitor has the general formula II: 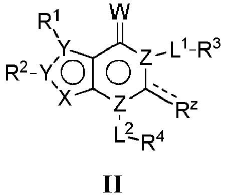 or a pharmaceutically acceptable salt thereof, wherein:
W is oxygen or sulfur;
X is O, S, N or NR;
each Y is independently C, N, or O;
each Z is independently C or N;
R¹ is hydrogen or C₁₋₄ aliphatic, optionally substituted with one or more halogens, -OR, -SR, - N(R)₂, -N(R)C(O)R, -C(O)N(R)₂, -N(R)C(O)N(R)₂, -N(R)C(O)OR, -OC(O)N(R)₂,-N(R)SO₂R, -SO₂RN(R)₂, -C(O)R, -C(O)OR, -OC(O)R, -C(O)OR, -S(O)R, or -SO₂R;
R² is halogen, -R, -OR, -SR, -N(R)₂, -N(R)C(O)R, - C(O)N(R)₂, -N(R)C(O)N(R)₂, -N(R)C(O)OR, -OC(O)N(R)₂, -N(R)SO₂R,-SO₂N(R)₂, -C(O)R, -C(O)OR, -OC(O)R, -S(O)R, -SO₂R, -B(OR)₂, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; wherein R² is not optionally substituted benzyl; or
R¹ and R² are taken together to form an optionally substituted 4-7 membered partially unsaturated carbocyclo-, orheterocyclo-, benzo-, or 5-6 memberedheteroarylo- fused ring;
each R is independently hydrogen, deuterium, or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1 -5 heteroatoms independently selected from nitrogen, oxygen, or sulfur;
L¹ is a covalent bond or a 1-6 membered straight or branched bivalent hydrocarbon chain optionally substituted with R⁵ and R^{5'};
L² is a covalent bond or a 1-6 membered straight or branched bivalent hydrocarbon chain optionally substituted with R⁷ and R^{7'};
R³ is halogen, -CN, -OR, -SR, -N(R)₂, -N(R)C(O)R, -C(O)RN(R)₂, -C(O)N(R)S(O)₂R, - N(R)C(O)N(R)₂, -N(R)C(O)OR, -OC(O)N(R)₂, -N(R)SO₂R, -SO₂N(R)₂, -C(O)R, -C(O)OR,-OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
each of R⁵ and R^{5'} is independently -R, -OR, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, or -SO₂R; or R⁵ and R^{5'} are taken together to form a cyclopropylenyl, cyclobutylenyl, or oxetanyl group;
each of R⁷ and R^{7'} is independently, -R, -OR⁶, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, -SO₂R, -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
R⁶ is -R, -C(O)N(R)₂, or -C(O)R;
each R⁸ is independently selected from halogen, -R, -OR, -SR, -N(R)₂ or deuterium;
R^{z} is selected from hydrogen, halogen, methyl, -CN, =O, and =S; and
n is 0-5.

6. The composition for the use according to any one of claims 1-3, wherein the ACC inhibitor has the general formula III: 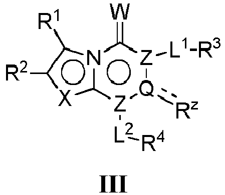 or a pharmaceutically acceptable salt thereof, wherein:
W is oxygen or sulfur;
Q is C or N; wherein if Q is N, then R^{z} is absent;
X is -O-, -S-, or -NR-;
each Z is independently C or N; provided that both Z are not N;
R¹ is hydrogen or C₁₋₄ aliphatic, optionally substituted with one or more halogens, -OR, -SR, - N(R)₂, -N(R)C(O)R, -C(O)N(R)₂, -N(R)C(O)N(R)₂, -N(R)C(O)OR, -OC(O)N(R)₂, - N(R)SO₂R, -SO₂RN(R)₂, -C(O)R, -C(O)OR, -OC(O)R, -C(O)OR, -S(O)R, or -SO₂R;
R² is halogen, -R, -OR, -SR, -N(R)₂, -N(R)C(O)R, -C(O)N(R)₂, -N(R)C(O)N(R)₂, - N(R)C(O)OR, -OC(O)N(R)₂, -N(R)SO₂R, -SO₂N(R)₂, -C(O)R, -C(O)OR, -OC(O)R, - S(O)R, -SO₂R, -B(OR)₂, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; or
R¹ and R² are taken together to form an optionally substituted 4-7 membered partially unsaturated carbocyclo-, orheterocyclo-, benzo-, or 5-6 memberedheteroarylo- fused ring;
each R is independently hydrogen, deuterium, or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
L¹ is a covalent bond or a 1-6 membered straight or branched bivalent hydrocarbon chain optionally substituted with R⁵ and R^{5'};
L² is a covalent bond or a 1-6 membered straight or branched bivalent hydrocarbon chain optionally substituted with R⁷ and R^{7'};
R³ is halogen, -CN, -OR, -SR, -N(R)₂, -N(R)C(O)R, -C(O)RN(R)₂, -C(O)N(R)S(O)₂R, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
each of R⁵ and R^{5'} is independently -R, -OR, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, or -SO₂R; or R⁵ and R^{5'} are taken together to form a cyclopropylenyl, cyclobutylenyl, or oxetanyl group; and
each of R⁷ and R^{7'} is independently, -R, -OR⁶, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, -SO₂R, -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
R⁶ is -R, -C(O)N(R)₂, or -C(O)R;
each R⁸ is independently selected from halogen, -R, -OR, -SR, -N(R)₂ or deuterium;
R^{z} is selected from hydrogen, halogen, methyl, -CN, =O, and =S; and
n is 0-5.

7. The composition for the use according to any one of claims 1-3, wherein the ACC inhibitor has the general formula IV: 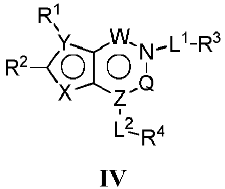 or a pharmaceutically acceptable salt thereof, wherein:
W is -C(O)-, -C(S)-, or -S(O)₂-;
Q is -C(O)-, -C(S)-, -S(O)₂-, or N;
X is -O-, -S-, -NR-, or N;
Y is C or N;
Z is C or N;
R¹ is hydrogen or C₁₋₄ aliphatic, optionally substituted with one or more halogens, -OR, -SR, - N(R)₂, -N(R)C(O)R, -C(O)N(R)₂, -N(R)C(O)N(R)₂, -N(R)C(O)OR, -OC(O)N(R)₂, - N(R)SO₂R, -SO₂RN(R)₂, -C(O)R, -C(O)OR, -OC(O)R, -C(O)OR, -S(O)R, or -SO₂R;
R² is halogen, -R, -OR, -SR, -N(R)₂, -N(R)C(O)R, - C(O)N(R)₂, -N(R)C(O)N(R)₂, -N(R)C(O)OR, -OC(O)N(R)₂, -N(R)SO₂R, - SO₂N(R)₂, -C(O)R, -C(O)OR, -OC(O)R, -S(O)R, or -SO₂R, -B(OR)₂, or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; wherein R² is not optionally substituted benzyl; or
R¹ and R² are taken together to form an optionally substituted 4-7 membered partially unsaturated carbocyclo-, orheterocyclo-, benzo-, or 5-6 memberedheteroarylo- fused ring;
each R is independently hydrogen, deuterium, or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
L¹ is a covalent bond or a 1-6 membered straight or branched bivalent hydrocarbon chain optionally substituted with R⁵ and R^{5'};
L² is a covalent bond or a 1-6 membered straight or branched bivalent hydrocarbon chain optionally substituted with R⁷ and R^{7'};
R³ is halogen, -CN, -OR, -SR, -N(R)₂, -N(R)C(O)R, -C(O)RN(R)₂, -C(O)N(R)S(O)₂R, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
each of R⁵ and R^{5'} is independently -R, -OR, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, or -SO₂R; or R⁵ and R^{5'} are taken together to form a cyclopropylenyl, cyclobutylenyl, or oxetanyl group;
each of R⁷ and R^{7'} is independently, -R, -OR⁶, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, -SO₂R, or -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
R⁶ is -R, -C(O)N(R)₂, or -C(O)R;
each R⁸ is independently selected from halogen, -R, -OR, -SR, -N(R)₂ or deuterium; and
n is 0-5.

8. The composition for the use according to any one of claims 1-3, wherein the ACC inhibitor has the formula V: 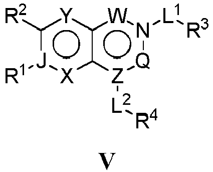 or a pharmaceutically acceptable salt thereof, wherein:
W is -C(R^{z})-, -C(O)-, or -C(S)-;
Q is -C(R^{z})-, -C(O)-, or -C(S)-;
J is C or N; provided that when J is N, R¹ is absent;
X is CH or N;
Y is CH or N;
Z is C or N;
R¹ is hydrogen or C₁₋₄ aliphatic, optionally substituted with one or more halogens, -OR, -SR, - N(R)₂, -N(R)C(O)R, -C(O)N(R)₂, -N(R)C(O)N(R)₂, -N(R)C(O)OR, -OC(O)N(R)₂, - N(R)SO₂R, -SO₂RN(R)₂, -C(O)R, -C(O)OR, -OC(O)R, -C(O)OR, -S(O)R, or -SO₂R;
R² is halogen, -R, -OR, -SR, -N(R)₂, -N(R)C(O)R, -C(O)N(R)₂, -N(R)C(O)N(R)₂, - N(R)C(O)OR, -OC(O)N(R)₂, -N(R)SO₂R, -SO₂N(R)₂, -C(O)R, -C(O)OR, -OC(O)R, - S(O)R, -B(OR)₂, -SO₂R or Hy, where Hy is selected from 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur; or
R¹ and R² are taken together to form an optionally substituted 4-7 membered partially unsaturated carbocyclo-, orheterocyclo-, benzo-, or 5-6 membered heteroarylo- fused ring;
each R is independently hydrogen, deuterium, or an optionally substituted group selected from C₁₋₆ aliphatic, a 3-8 membered saturated or partially unsaturated monocyclic carbocyclic ring, phenyl, an 8-10 membered bicyclic aromatic carbocyclic ring; a 4-8 membered saturated or partially unsaturated monocyclic heterocyclic ring having 1-2 heteroatoms independently selected from nitrogen, oxygen, or sulfur, a 5-6 membered monocyclic heteroaromatic ring having 1-4 heteroatoms independently selected from nitrogen, oxygen, or sulfur, or an 8-10 membered bicyclic heteroaromatic ring having 1-5 heteroatoms independently selected from nitrogen, oxygen, or sulfur;
L¹ is a covalent bond or a 1-6 membered straight or branched bivalent hydrocarbon chain optionally substituted with R⁵ and R^{5'};
L² is a covalent bond or a 1-6 membered straight or branched bivalent hydrocarbon chain optionally substituted with R⁷ and R^{7'};
R³ is hydrogen, halogen, -CN, -OR, -SR, -N(R)₂, -N(R)C(O)R, -C(O)RN(R)₂, -C(O)N(R)S(O)₂R, -N(R)C(O)N(R)₂, -N(R)C(O)OR, -OC(O)N(R)₂, -N(R)SO₂R, -SO₂N(R)₂, -C(O)R, -C(O)OR, -OC(O)R, -S(O)R, -SO₂R, -B(OR)₂, or an optionally substituted ring selected from phenyl or 5-6 membered heteroaryl having 1-4 heteroatoms independently selected from nitrogen, oxygen, or sulfur;
R⁴ is hydrogen or a ring selected from a 3-8 membered monocyclic saturated or partially unsaturated carbocyclic ring, a 4-8 membered monocyclic saturated or partially unsaturated heterocyclic ring having 1-2 heteroatoms independently selected from nitrogen, oxygen, or sulfur, phenyl, an 8-10 membered bicyclic aryl ring, a 5-6 membered monocyclic heteroaryl ring having 1-4 heteroatoms independently selected from nitrogen, oxygen, or sulfur, or an 8-10 membered bicyclic heteroaryl ring having 1-4 heteroatoms independently selected from nitrogen, oxygen, or sulfur; wherein said ring is optionally substituted with n instances of R⁸;
each of R⁵ and R⁵' is independently -R, -OR, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, or -SO₂R; or R⁵ and R^{5'} are taken together to form a cyclopropylenyl, cyclobutylenyl, or oxetanyl group;
each of R⁷ and R^{7'} is independently, -R, -OR⁶, -SR, -N(R)₂, -N(R)C(O)R, -C(O)N(R)₂, - N(R)C(O)N(R)₂, -N(R)C(O)OR, -OC(O)N(R)₂, -N(R)SO₂R, -SO₂N(R)₂, -C(O)R, -C(O)OR, - OC(O)R, -S(O)R, -SO₂R, or -B(OR)₂; or R⁷ and R^{7'} are taken together to form a 3-8 membered saturated or partially unsaturated monocyclic carbocyclic ring, or a 4-8 membered saturated or partially unsaturated monocyclic heterocyclic ring having 1-2 heteroatoms independently selected from nitrogen, oxygen, or sulfur;
R⁶ is -R, -C(O)N(R)₂, or -C(O)R;
each R⁸ is independently selected from halogen, -R, -OR, -SR, -N(R)₂ or deuterium;
each R^{z} is independently selected from the group consisting of hydrogen, halogen, C₁-C₃ alkyl, and -CN; and
n is 0-5.

9. The composition for the use according to any one of claims 1 to 8 wherein the ACC inhibitor is administered in combination with one or more additional therapeutic agents; and wherein:
the ACC inhibitor and the one or more additional therapeutic agents are administered simultaneously, or
the ACC inhibitor and the one or more additional therapeutic agents are administered sequentially.

10. The composition for the use according to any one of claims 1-9, wherein:
the non-alcoholic fatty liver disease is steatosis;
the non-alcoholic fatty liver disease is non-alcoholic steatohepatitis (NASH);
the non-alcoholic fatty liver disease is liver fibrosis caused by NASH;
the non-alcoholic fatty liver disease is liver cirrhosis caused by NASH; or
the non-alcoholic fatty liver disease is hepatocellular carcinoma (HCC) caused by NASH.

11. A combination for use in treating, stabilizing or lessening the severity of a non-alcoholic fatty liver disease, the combination comprising an ACC inhibitor, in combination with one or more additional therapeutic agents.

12. The combination for the use according to claim 11, wherein at least one of the one or more additional therapeutic agents is:
an anti-diabetic agent, such as an adenosine A₁ receptor agonist (e.g. adenosine, CCPA, CVT-3619, GR-190718), an adenosine A₂ receptor antagonist (e.g. istradefylline, SCH-58261), an aldose reductase inhibitor, an α-amylase inhibitor (e.g. tendamistat, treastatin, AL-3688), an α-glucosidase inhibitor (e.g. acarbose, camiglibose, diposine, emiglitate, miglitol, pradimicin-Q, sarbostatin, voglibose), an amylin analog (e.g. AC 164209 and pramlintide), an AMPK activator, a β3-adrenergic agonist (e.g. amibegron, AZ-40140, CL-316,243, KRP-204, L-742,791, L-796,568, LY-368,842, LY-377,604, mirabegron, Ro 40-2148, solabegron, SWR-0342SA), a β-ketoacyl-acyl carrier protein synthase inhibitor, a biguanide (e.g. metformin, buformin, phenformin), a carnitine palmitoyl transferase inhibitor, a DGAT-2 inhibitor, a DPP-4 inhibitor (e.g. alogliptin, anagliptin, dutogliptin, gemigliptin, linagliptin, omarigliptin, saxagliptin, sitagliptin, teneligliptin, trelagliptin, and vildagliptin), an ERN1 inhibitor, a fatty acid oxidation inhibitor, a fatty acid synthase (FAS) inhibitor, an FGF21 derivative, a fructose 1,6-diphosphatase inhibitor, a GLP1 agonist (e.g. albiglutide, dulaglutide, exenatide, liraglutide, lixisenatide, taspoglutide), a glucagon receptor modulator, a mixed glucagon receptor / GLP-1 agonist (e.g. MAR-701, ZP2929), a glucokinase inhibitor (e.g. TTP-399, TTP-355, TTP-547, AZD1656, ARRY403, MK-0599, TAK-329, AZD5658, and GKM-001), a glycogen phosphorylase inhibitor (e.g. GSK1362885), a GSK-3 inhibitor, a GPR119 agonist (e.g. MBX-2982, GSK1292263, APD597, PSN821), a GPBAR1 (TGR5) agonist (e.g. INT-777, XL-475), a GPR39 modulator, a GPR40 agonist (e.g. TAK-875), a GPR41 modulator, a GPR43 modulator, a GPR81 modulator, a GPR120 agonist, an HSL inhibitor, an IκB inhibitor, an ILI-beta modulator, insulin or an insulin analog (including, but not limited to, oral, inhaled or injectable formulations thereof), insulin-like growth factor (IGF-1) or an analog thereof, an insulin secretagogue, a JNK inhibitor (e.g. CC-359), a kappa opioid receptor modulator, LY3084077, a Kvl.3 inhibitor (e.g. ChTX, clofazmine, WIN-173173), a MAP4K4 inhibitor, an MC₁ or MC₄ agonist (e.g. afamelanotide, BMS-470539, bremelanotide, Melanotan II, PF-00446687, PL-6983, setmelanotide, and THIQ), a meglitinide (e.g. repaglinide, nateglinide, mitiglinide), a mineralocorticoid receptor inhibitor, a monoacylglycerol O-acyltransferase inhibitor, an NF-κB inhibitor, a nicotinic acid receptor (HM74A) activator, a PDE-10 inhibitor, a PDHK2 inhibitor, a PDHK4 inhibitor, a PKC (including PKC-alpha, PKC-beta, and PKC-gamma) inhibitor, a PPARα/γ dual agonist, a PTP1b inhibitor (e.g. trodusquemine), a retinol binding protein 4 inhibitor, a serine palmitoyl transferase inhibitor, an SGLT1 inhibitor (e.g. GSK1614235), a SIRT-1 inhibitor (e.g. resveratrol, GSK2245840, GSK184072), a somatostatin receptor inhibitor, a sulfonylurea (e.g. acetohexamide, chlorpropamide, diabinese, glibenclamide, glipizide, glyburide, blimipiride, gliclazide, glipentide, gliquidone, glisolamide, tolazamide, tolbutamide), a thiazolidinedione (e.g. ciglitazone, darglitazone, englitazone, lobeglitazone, MSDC-0602, netoglitazone, pioglitazone, rivoglitazone, rosiglitazone, and troglitazone), a TORC2 inhibitor, a urotensin II receptor agonist, a vasopressin agonist (e.g. DDAVP, WAY-141608), or a VPAC2 receptor agonist; or
an anti-obesity agent, such as an apoB-MTP inhibitor (e.g. dirlotapide, JTT130, SLX4090, usistapide), a β3-adrenergic agonist (e.g. amibegron, AZ-40140, CL-316,243, KRP-204, L-742,791, L-796,568, LY-368,842, LY-377,604, mirabegron, Ro 40-2148, solabegron, SWR-0342SA), a bombesin receptor agonist, a BRS3 modulator, a CB1 receptor antagonist or inverse agonist, a CCK_{A} agonist, ciliary neurotrophic factor (CNTF) or analog thereof (e.g. axokine, NT-501), buproprion/naltrexone, a dopamine receptor agonist (e.g. bromocriptine), an 11β-hydroxysteroid dehydrogenase (11β-HSD1) inhibitor, pramlintide/metreleptin, a 5-HT_{2C} agonist (e.g. lorcaserin), a galanin antagonist, a ghrelin agonist or antagonist, a GLP1 agonist (e.g. albiglutide, dulaglutide, exenatide, liraglutide, lixisenatide, taspoglutide), a mixed glucagon receptor / GLP-1 agonist (e.g. MAR-701, ZP2929), an H3 antagonist or inverse agonist, a human agouti-related protein (AGRP) inhibitor, leptin or an analog thereof (e.g. metreleptin), a lipase inhibitor (e.g. tetrahydrolipstatin), an MC₁ or MC₄ agonist (e.g. afamelanotide, BMS-470539, bremelanotide, Melanotan II, PF-00446687, PL-6983, setmelanotide, and THIQ), a melanocyte-stimulating hormone or analog thereof, a MetAp2 inhibitor (e.g. ZGN-433), a monoamine reuptake inhibitor (e.g. buproprion, sibutramine, phentermine, tesofensine), a neuromedin U receptor agonist, an NPY antagonist (e.g. velneperit), an opioid receptor antagonist (e.g. naltrexone), an orexin receptor antagonist (e.g. almorexant, lemborexant, SB-334,867, SB-408,124, SB-649,868, suvorexant), oxyntomodulin or an analog thereof, PYY or an analog thereof (e.g. PYY₁₋₃₆, PYY₃₋₃₆), phentermine/topiramate, an RXR-alpha modulator, a stearoyl-CoA desaturase (SCD-1) inhibitor, or a sympathomimetic agent; or
an agent for treating a metabolic disorder, such as an ABC transporter activator, ACT-434964 (Actelion), an ANG-5 inhibitor, an angiotensin II antagonist (e.g. MC4262), CCX-872, DUR-928 (Durect), ESP41091, F-652 (Generon), an FGF21 agonist (e.g. BMS-986036), fomepizole (Raptor), an FXR agonist, dual FXR/TGR5 agonist (e.g. INT-767), a ghrelin antagonist (e.g. TZP-301), a glucosylceramide synthase inhibitor, a GPR17 modulator, a GPR119 agonist, IG-MD-014 (Indigene), IMM-124E (Immuron), a lysosome pathway modulator (e.g. CAT5000), a melanin-concentrating hormone receptor 1 antagonist (e.g. KI-1361-17), an MCL1 inhibitor (e.g. CMPX-1023), an mTORC1 inhibitor, an NaCT (e.g. SLC13A5) inhibitor, a NHE3 inhibitor (e.g. RDX-011, tenapanor), NP003 (Neuraltus), PBI-4050 (ProMetic), a proteostasis regulator (e.g. PTI-130, PTI-428, PTI-C1811), PS248288 (Pharmacopeia/Merck), PX-102 (Phenex), RG7410. RG7652, a ROCK inhibitor, SBC-104 (Synageva BioPharma), SPX-100 (Spherix), a stearoyl CoA desaturase inhibitor (e.g. CVT-12805), TRC150094 (Torrent), or ZYH7 (Zydus Cadila); or
an agent for treating steatosis, such as an adiponectin analog (e.g. PX 811013), aramchol (Galmed), an ASK1 inhibitor (e.g. GS4977, GS4997), AZD4076 (AstraZeneca), a bile acid sequestrant (e.g. obeticholic acid), BL-1060 (Galmed), BMS986171 (Bristol-Myers Squibb), a CCR5/CCR2 antagonist (e.g. cenicriviroc), cannabidiol, CER-209 (Cerenis), cysteamine analog (e.g. RP-103, RP-104), DS102 (DS Biopharma), EGS21 (Enzo), elafibranor (Genfit), emricasan (Idun), ethyl eicosapentaenoic acid (Mochida), an FXR agonist, a GPBAR1 agonist (e.g. RDX009), GR-MD-02 (Galectin Therapeutics), leucine/sildenafil/metformin (NuSirt), LCQ908 (Novartis), LJN452 (Novartis), a LOXL2 inhibitor (e.g. simtuzumab), MAT-8800 (Matinas), MB-10866 (Metabasis), an miR-103/107 inhibitor (e.g. RG-125), MK-4074 (Merck & Co.), nalmefene (TaiwanJ), nivocasan (Gilead), NGM-282 (NGM Biopharmaceuticals), an omega-3 carboxylic acid or mixture of the same, PX-102 (Phenex), PX-104 (Phenex), remogliflozin etabonate (Kissei), saroglitazar (Zydus-Cadila), SAR-548304 (sanofi-aventis), tipelukast (Kyorin), ursodeoxycholic acid, VK2809 (Viking), or XL335 (Exelixis); or
an agent for treating inflammation, such as agent for treating inflammation that reduces the differentiation or activation of Tₕ17 cells or that is a caspase inhibitor (e.g. emricasan), a TGF-β inhibitor, an IL-1β inhibitor, an IL-6 inhibitor, an IL-17 inhibitor, an IL-17a inhibitor, an IL-17F inhibitor, an IL-21 inhibitor, an IL-23 inhibitor (e.g. guselkumab), IMM-124E (Immuron), a RORyt inhibitor (e.g. JTE-151), a RORα inhibitor, solithromycin (Cempra), or a vascular adhesion protein-1 inhibitor (e.g. PXS-4728A); or
an agent for treating fibrosis, such as CNX-014/023/024/025 (Connexios), evitar (AdeTherapeutics), a galectin-3 inhibitor, IVA337 (Inventiva), pirfenidone, RG6069 (Roche), SP20102 (Sarfez), or XOMA 089 (Xoma).

13. The combination according to claim 11, wherein the one or more additional therapeutic agents are independently selected from the group consisting of angiotensin II receptor antagonists, angiotensin converting enzyme (ACE) inhibitors, caspase inhibitors, cathepsin B inhibitors, CCR2 chemokine antagonists, CCR5 chemokine antagonists, chloride channel stimulators, cholesterol solubilizers, diacylglycerol O-acyltransferase 1 (DGAT1) inhibitors, dipeptidyl peptidase IV (DPPIV) inhibitors, farnesoid X receptor (FXR) agonists, galectin-3 inhibitors, glucagon-like peptide 1 (GLP1) agonists, glutathione precursors, hepatitis C virus NS3 protease inhibitors, HMG CoA reductase inhibitors, 11β-hydroxysteroid dehydrogenase (11β-HSD1) inhibitors, IL-1β antagonists, IL-6 antagonists, IL-10 agonists, IL-17 antagonists, ileal sodium bile acid cotransporter inhibitors, leptin analogs, 5-lipoxygenase inhibitors, LPL gene stimulators, lysyl oxidase homolog 2 (LOXL2) inhibitors, PDE3 inhibitors, PDE4 inhibitors, phospholipase C (PLC) inhibitors, PPARα agonists, PPARγ agonists, PPARδ agonists, Rho associated protein kinase 2 (ROCK2) inhibitors, sodium glucose transporter-2 (SGLT2) inhibitors, stearoyl CoA desaturase-1 inhibitors, thyroid hormone receptor β agonists, tumor necrosis factor α (TNFα) ligand inhibitors, transglutaminase inhibitors, transglutaminase inhibitor precursors, PTPlb inhibitors, and ASK1 inhibitors; or
wherein the one or more additional therapeutic agents are independently selected from acetylsalicylic acid, alipogene tiparvovec, aramchol, atorvastatin, BLX-1002, cenicriviroc, cobiprostone, colesevelam, emricasan, enalapril, GFT-505, GR-MD-02, hydrochlorothiazide, icosapent ethyl ester (ethyl eicosapentaenoic acid), IMM-124E, KD-025, linagliptin, liraglutide, mercaptamine, MGL-3196, obeticholic acid, olesoxime, peg-ilodecakin, pioglitazone, PX-102, remogliflozin etabonate, SHP-626, solithromycin, tipelukast, TRX-318, ursodeoxycholic acid, and VBY-376.

14. The combination according to any one of claims 11-13, wherein the ACC inhibitor is: 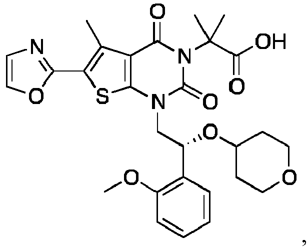 or
or a pharmaceutically acceptable salt thereof, or 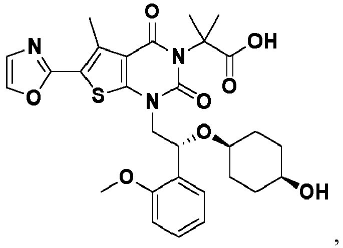 or a pharmaceutically acceptable salt thereof.

15. A composition comprising an ACC inhibitor, one or more additional therapeutic agents, and a pharmaceutically acceptable carrier, adjuvant, or vehicle.
